# FASCICULE DE BREVET EUROPEEN

(11) **EP 2 903 697 B1**
(45) Date de publication et mention de la délivrance du brevet: **27.11.2019**
(21) Numéro de dépôt: 13782748.1
(22) Date de dépôt: 24.09.2013
(51) Int. Cl.: C07C 29/40, C07C 67/08, C07C 35/18, C07C 35/21, C07C 69/145, C07C 69/157, C07C 45/62, C07C 45/68, C07C 45/74, C07C 49/403, C11B 9/00, C07C 29/143, C07C 41/16, C07C 43/188, C07C 49/603, C07C 49/613, C07C 49/653, C07C 45/61

(54) **PROCÉDÉ DE SYNTHÈSE DE CYCLOHEXÈNONES AINSI QUE LEUR UTILISATION EN PARFUMERIE**
VERFAHREN ZUR SYNTHESE VON CYCLOHEXENONEN UND VERWENDUNG DAVON IN DER PARFÜMINDUSTRIE
METHOD FOR SYNTHESISING CYCLOHEXENONES AND THE USE OF SAME IN THE PERFUME INDUSTRY

(30) Priorité: 05.10.2012 FR 1259524
(43) Date de publication de la demande: 12.08.2015
(73) Titulaire: V. Mane Fils, 06620 Le Bar-sur-Loup (FR)
(72) Inventeur: CHANOT, Jean-Jacques, 06530 Speracedes (FR); PLESSIS, Caroline, 06740 Chateauneuf (FR)
(74) Mandataire: Murgitroyd & Company
(86) Numéro de dépôt international: PCT/FR2013/052235
(87) Numéro de publication internationale: WO 2014/053744

(56) Documents cités:
- JP-A- 2008 127 333
- DATABASE CA [Online] CHEMICAL ABSTRACTS SERVICE, COLUMBUS, OHIO, US; KHEIFITS, L. A. ET AL: "Preparation of new perfumes from products of oxidation of camphene with 2,6-xylenol", XP002697648, extrait de STN Database accession no. 1962:423568 & KHEIFITS, L. A. ET AL: "Preparation of new perfumes from products of oxidation of camphene with 2,6-xylenol", ZHURNAL VSESOYUZNOGO KHIMICHESKOGO OBSHCHESTVA IM. D. I. MENDELEEVA , 7, 234-5 CODEN: ZVKOA6; ISSN: 0373-0247, 1962,
- DATABASE CA [Online] CHEMICAL ABSTRACTS SERVICE, COLUMBUS, OHIO, US; KHEIFITS, L. A. ET AL: "Relation between structure and odor in several norbornylcyclanones", XP002697649, extrait de STN Database accession no. 1967:65168 & KHEIFITS, L. A. ET AL: "Relation between structure and odor in several norbornylcyclanones", TRUDY VSESOYUZNOGO NAUCHNO-ISSLEDOVATEL'SKOGO INSTITUTA SINTETICHESKIKH I NATURAL'NYKH DUSHISTYKH VESHCHESTV , NO. 7, 63-9 FROM: REF. ZH. KHIM. 1966, PT. II, ABSTR. NO. 20R449 CODEN: TVDVAS; ISSN: 0463-5833, 1965,
- DATABASE CA [Online] CHEMICAL ABSTRACTS SERVICE, COLUMBUS, OHIO, US; GURA, YU. ET AL: "Terpene phenols. XIX. Condensations of norbornane with xylenols and further conversions of the reaction products", XP002697650, extrait de STN Database accession no. 1966:44004 & GURA, YU. ET AL: "Terpene phenols. XIX. Condensations of norbornane with xylenols and further conversions of the reaction products", PROBL. ORGAN. SINTEZA, AKAD. NAUK SSSR, OTD. OBSHCH. I. TEKHN. KHIM. 91-100, 1965,
- KHEJFIK: PARFUMERIE, COSMETIQUE, SAVONS, SN, PARIS, FR, vol. 8, no. 8, 1 August 1965 (1965-08-01), pages 342-349, XP009505149, ISSN: 0369-9099

## Description

La présente invention concerne un nouveau procédé simple, peu coûteux et sûr, de synthèse de composés cyclohexènones et cyclohexènols, lesdits composés pouvant être utilisés dans l'industrie de la parfumerie, de la cosmétique, et des détergents notamment, lesdits composés présentant des fragrances et des propriétés de rémanence particulières.

Les cyclohexènones ainsi que les cyclohexènols sont des familles de composés importantes dans l'industrie du parfum et des arômes. De nombreux composés appartenant à ces familles ont été décrits et synthétisés, comme par exemple la Celery Ketone, ou encore les composés décrits dans les brevets EP 0504592 (Firmenich) ou encore JP 2008127333 (Kao Corp). Cependant, pour ces derniers, un des problèmes techniques concernant ce type de composés réside dans leur procédé de synthèse. Par exemple, le brevet EP 0504592 décrit un procédé de synthèse de cyclohexènones par condensation entre une alkyl vinyl cétone et un dérivé carbonylé. Ce procédé présente des inconvénients, notamment en ce qu'il est difficile à mettre en œuvre, que les réactions qui ont lieu sont instables et qu'il a un coût important (du fait du coût élevé des produits de départ). Mais le problème principal de ce procédé réside dans sa dangerosité lors de sa mise en pratique à cause de l'alkyl vinyl cétone. Une autre méthode qui a été mise en œuvre dans l'art antérieur consiste à faire réagir un β-cétoester avec un méthylène-aldéhyde. Ce type de procédé est notamment décrit dans la demande de brevet JP 2008127333. Mais là encore, les substrats de départ ont un coût élevé (notamment le β-cétoester qu'il faut synthétiser avec différents substituants) et surtout le procédé est difficilement industrialisable. Or, l'industrie des parfums et arômes a un besoin constant de trouver de nouvelles molécules afin de faire face au mieux à un nombre croissant de contraintes (contraintes environnementales, réglementaires, économiques...).

A cet effet, afin de pouvoir obtenir aisément de nouveaux composés fragrants et/ou aromatiques, la Demanderesse a développé un nouveau procédé de synthèse de composés cyclohexènones et cyclohexènols. Non seulement le procédé en question est nouveau et inventif, mais il permet aussi d'obtenir un nombre important de composés eux-mêmes nouveaux et inventifs outre des composés déjà connus dans l'art antérieur. Le procédé selon la présente invention consiste en la condensation d'une cétone sur un α-méthylène-aldéhyde afin d'obtenir par une réaction domino, des composés cyclohexènones de formule (I) suivante : 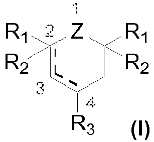 dans laquelle :
- R1 représente un méthyle ou un éthyle ;
- R2 représente indépendamment un hydrogène ou un groupement C1-C5 alkyle ou C2-C5 alcényle;
- R3 représente un groupement alkyle ou alcényle, éventuellement substitué par un aryle, ou R3 représente un groupement alkyle cyclique ou alcényle cyclique, éventuellement substitué par un ou plusieurs groupements C1-C6 alkyle, étant entendu que R3 comporte au total 3 à 10 atomes de carbone;
- Z représente C(O) ou CR4(OR5), avec
   - R4 représente un hydrogène ou un groupement C1-C8 alkyle ou C2-C8 alcényle ;
   - R5 représente un hydrogène ou un groupement C1-C8 alkyle ou alcanoyle, ou C2-C8 alcényle ou alcénoyle ;
   sachant qu'une double liaison est présente ou absente, sur le cycle et que lorsqu'elle est présente, elle est
   - soit en position 2-3 et R2 est absent en position 2,
   - soit en position 3-4 et R2 est présent en position 2 et est tel que défini précédemment.

A la connaissance de la Demanderesse, ce type de réaction n'a jamais été décrit auparavant. A l'issue de cette première étape de réaction, il est possible d'obtenir différents types de dérivés cyclohexènones, ainsi que des composés cyclohexènols. Le procédé selon la présente invention est nouveau et inventif par rapport, notamment à celui décrit dans le brevet US4326997 dans lequel des composés présentant des structures proches sont décrits. En outre, le procédé décrit dans ledit brevet ne permet pas l'obtention de tous les composés de formule (I) alors que le présent procédé le permet. Enfin, le procédé selon la présente invention a l'avantage d'être simple, peu coûteux (par l'utilisation de substrats bon marché), sans risques, et d'être facilement industrialisable et de présenter un bon rendement.

La présente invention a également pour objet des composés ainsi que leur utilisation en parfumerie, caractérisé en ce que lesdits composés répondent à la formule générale (II) suivante : 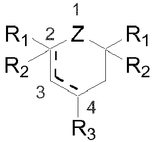 dans laquelle :
- R1 représente un méthyle ou un éthyle ;
- R2 représente indépendamment un hydrogène ou un groupement C1-C5 alkyle ou C2-C5 alcényle ;
- R3 représente un groupement alkyle ou alcényle, éventuellement substitué par un aryle, ou R3 représente un groupement alkyle cyclique comprenant cinq à six atomes de carbone ou alcényle cyclique, éventuellement substitué par un ou plusieurs groupements C1-C6 alkyle, étant entendu que R3 comporte au total 7 à 10 atomes de carbone ;
- Z représente C(O) ou CR4(OR5), avec
   - R4 représente un hydrogène ou un groupement C1-C8 alkyle ou C2-C8 alcényle ;
   - R5 représente un hydrogène ou un groupement C1-C8 alkyle ou alcanoyle, ou C2-C8 alcényle ou alcénoyle ;
   sachant qu'une double liaison est présente ou absente, sur le cycle et que lorsqu'elle est présente, elle est
   - soit en position 2-3 et R2 est absent en position 2,
   - soit en position 3-4 et R2 est présent en position 2 et est tel que défini précédemment;
   ledit composé étant sous forme d'un stéréoisomère ou d'un mélange de stéréoisomères, ou d'un mélange racémique.

Le brevet US 4326997 décrit des composés fragrants proches de la formule générale (II), notamment des composés cyclohexènones et cyclohexènols comportant un groupement R3=(CH2)2-i-Pr et des descriptions olfactives suivantes : balsamique, boisé, doux, terreux, herbacé... descriptions qui sont différentes de celles des composés revendiqués présentement.

Des composés proches de ceux couverts par la susdite formule générale (II) sont également divulgués au sein des publications suivantes :
i) KHEIFITS, L. A. ET AL. « Préparation of new perfumes from products of oxidation of camphene with 2,6-xylenol », Zhurnal Vsesoyuznogo Khimicheskogo Obshchestva im. D.I. Mendeleeva, (1962), 7, 234-5,
ii) KHEIFITS, L. A. ET AL. « Relation between structure and odor in several norbornylcyclanones », Trudy Vsesoyuznogo Nauchno-Issledovatel'skogo Instituta Sinteticheskikh i Natural'nykh Dushistykh Veshchestv, (1965), No. 7, 63-9 (*from: Ref.* ZH. KHim. (1966), Pt. II, ABstr.No. 20R449*),* et
iii) GURA, YU. ET AL. « Terpene phénols. XIX. Condensations of norbornane with xylenols and further conversions of the reaction products », Probl. Organ. Sinteza, Akad. Nauk SSSR, Otd. Obshch. i. Tekhn. Khim, (1965), 91-100*.*

Toutefois, ces composés comprennent, en tant que groupement R3, un groupement alkyle bi-cyclique ponté à sept atomes de carbone, à savoir un groupement isobornyle (groupement alkyle bi-cyclique ponté à sept atomes de carbone substitué par trois groupements méthyle ; cf. publication mentionnée au point i) supra) ou norbonyle (se distinguant du susdit groupement isobornyle par l'absence des trois groupements méthyle ; cf. publications mentionnées aux points ii) et iii) supra).

Une publication scientifique de L.A. Khejfik et al. (Parf. Cosm. Sav. vol.8, N°8, août 1965) décrit des composés cyclohexènones et cyclohexènols comportant, en tant que groupement R3, un groupement alkyle saturé ayant 6 atomes de carbones. Cependant, ces composés sont décrits comme présentant des notes iris-ionone et pas des notes santalées, boisées ou vertes comme c'est le cas pour les dérivés de l'invention.

Un troisième objet de la présente invention se rapporte à des compositions comprenant des composés de formule (II).

Un dernier objet de la présente invention se rapporte à l'utilisation de composés de formule (III) suivante : 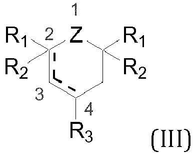 dans laquelle :
- R1 représente un méthyle ou un éthyle ;
- R2 représente indépendamment un hydrogène ou un groupement C1-C5 alkyle ou C2-C5 alcényle ;
- R3 représente un groupement alkyle ou alcényle, éventuellement substitué par un aryle, ou R3 représente un groupement alkyle cyclique comprenant cinq à six atomes de carbone ou alcényle cyclique, éventuellement substitué par un ou plusieurs groupements C1-C6 alkyle, étant
entendu que R3 comporte au total 3 à 10 atomes de carbone et qu'il comporte au moins une insaturation lorsqu'il compte 5 à 6 atomes de carbone ;
- Z représente C(O) ou CR4(OR5), avec
   - R4 représente un hydrogène ou un groupement C1-C8 alkyle ou C2-C8 alcényle ;
   - R5 représente un hydrogène ou un groupement C1-C8 alkyle ou alcanoyle, ou C2-C8 alcényle ou alcénoyle ;
   sachant qu'une double liaison est présente ou absente, sur le cycle et que lorsqu'elle est présente, elle est
   - soit en position 2-3 et R2 est absent en position 2,
   - soit en position 3-4 et R2 est présent en position 2 et est tel que défini précédemment.

Le terme « alkyle » au sens de la présente invention désigne un groupe hydrocarboné saturé linéaire ou ramifié, ayant de préférence de 1 à 10 atomes de carbone. Comme exemples de groupe alkyle, on peut citer notamment les groupes méthyle, éthyle, n-propyle, *i*-propyle, *n*-butyle, *i*-butyle, *t*-butyle, pentyle et hexyle.

L'expression « alkyle cyclique » désigne un groupe hydrocarboné saturé cyclique, ayant de préférence de 3 à 10 atomes de carbone. De préférence, le groupe comprend 5 à 6 atomes de carbone, et encore plus préférentiellement 5 atomes de carbone Comme exemples de groupes alkyle cyclique, on peut citer notamment le cyclopentyle.

Le terme « alcényle », au sens de la présente invention, désigne un groupe hydrocarboné insaturé linéaire ou ramifié contenant au moins une double liaison carbone-carbone et ayant de préférence de 2 à 10 atomes de carbone. Comme exemples de groupe alcényle, on peut citer notamment les groupes vinyle, allyle, méthallyle, 2-propényle, isopropényle, 2-butényle, 3-butényle, 2-pentényle, 3-pentényle, 4-pentényle, 1-hexényle, etc.

L'expression « alcényle cyclique » désigne un groupe hydrocarboné insaturé cyclique, ayant de préférence de 3 à 10 atomes de carbone. Comme exemples de groupes alcényle cyclique, on peut citer notamment le cyclopentényle.

Le terme « alcanoyle », au sens de la présente invention, désigne un groupe - C(O)-alkyl, dérivé d'un acide carboxylique, dans lequel le groupe alkyle est tel que défini ci-dessus. Comme exemples de groupe alcanoyle, on peut citer notamment les groupements formyl, acétyl, propionyl, butyryl, etc.

Le terme « alcènoyle », au sens de la présente invention, désigne un groupe - C(O)-alcényl, dérivé d'un acide carboxylique, dans lequel le groupe alcènyle est tel que défini ci-dessus. Comme exemples de groupe alcénoyle, on peut citer notamment 2-propènoyl, 2-butènoyl, 2-méthyl-2-propènoyl, etc.

Le terme « aryle » désigne un groupe fonctionnel hydrocarboné dérivant d'un hydrocarbure aromatique ayant de 6 à 14 atomes de carbone, de préférence de 6 à 10 atomes de carbone. Comme exemples de groupe aryle, on peut citer notamment les groupements phényl, tolyl, naphtyl, xylyl.

Un premier objet de la présente invention se rapporte donc à un procédé de préparation d'un composé de formule (I) tel que décrit précédemment comprenant les étapes suivantes :
i) réaction d'un α-méthylène-aldéhyde, en présence d'une base, sur une cétone symétrique pour obtenir un composé de formule (la) dans laquelle R1, et R3 sont tels que définis précédemment, R2 est un hydrogène, et une double liaison en 2-3 ou 3-4 est présente sur le cycle et, cette réaction étant suivie éventuellement par les étapes ii), et/ou iii), et/ou iv),
ii) réaction de mono- ou bis-alkylation afin d'obtenir un composé de formule (la) dans laquelle R2 est un groupement C1-C5 alkyle ou C2-C5 alcényle ;
iii) conversion de la fonction Z = C(O) du composé obtenu à l'étape précédente en une fonction Z=CR4(OR5), R4 et R5 étant tels que définis précédemment;
iv) réduction de la double liaison en 2-3 ou 3-4 présente sur le cycle du composé obtenu à l'étape précédente, l'étape iv) pouvant être réalisée après l'une quelconque des étapes i), ii), ou iii).

Le schéma ci-dessous représente l'étape i) du procédé tel que décrit ci-dessus. 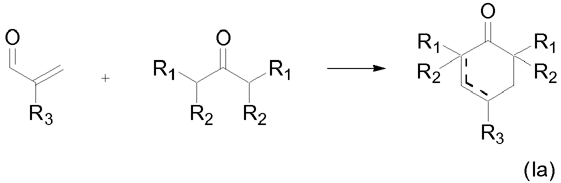 A la connaissance de la Demanderesse, ce type de réaction n'a jamais été reporté.

Seules des réactions domino de type annélation de Robinson entre un dérivé carbonylé et la méthyl vinyl cétone (EP 0504592 Firmenich) ou encore des réactions à l'aide d'un β-cétoester (JP 2008127333) ont été décrites.
L'étape i) entre l'a-méthylène-aldéhyde et une cétone symétrique se fait en présence d'une base. La base utilisable peut être choisie parmi les bases inorganiques (KOH, NaOH, LiOH ...) ou les bases organométalliques (*t*-Bu-OK, MeONa...). Les solvants utilisables dans la présente réaction sont notamment l'eau, l'éthanol, le méthanol, le toluène, le cyclohexane ou le THF... en mélange ou non. De préférence, on utilisera un mélange eau/éthanol. Cette étape i) de réaction peut être réalisée à température ambiante à reflux du solvant, de préférence entre 50 et 70°C.

Suite à la formation du composé de formule (Ia) à l'issue de l'étape i), une étape supplémentaire optionnelle ii), permet d'obtenir des composés de formule (Ia) tel que décrit précédemment, dans laquelle R2 est un groupement C1-C5 alkyle ou C2-C5 alcényle. Une première étape ii) de mono-alkylation permet d'obtenir un composé de formule (Ia) ayant un groupement R2 alkylé ou alcénylé en position 6 du cycle, la double liaison 2-3 étant présente. Les mêmes composés peuvent être alkylés une seconde fois afin de donner des composés de formule (Ia) où R2 est un alkyle ou un alcényle au niveau des positions 2 et 6 du cycle ; dans ce cas la double liaison du cycle est en position 3-4 (les groupements R1 et R3 étant définis comme précédemment).

L'étape ii) est suivie éventuellement par une étape iii) de conversion d'un composé de formule (Ia) obtenu à l'issue des étapes i), ii) ou iv) en un composé de formule (I) dans laquelle R1, R2 et R3 sont tels que définis précédemment, et Z=CR4(OR5). Le procédé selon l'invention comprend éventuellement une étape iv) de réduction de la double liaison présente sur le cycle en 2-3 ou 3-4 du composé obtenu à l'étape précédente, l'étape iv) pouvant être réalisée après l'une quelconque des étapes i), ii) ou iii). Les paragraphes suivants détaillent les différentes étapes permettant l'obtention des composés de formule (I).

Dans un premier mode de réalisation de l'invention, l'étape iii) comprend une étape iii.a) de réduction de la fonction cétone du composé de formule (Ia) obtenu aux étapes i), ii) ou iv) afin d'obtenir un composé de formule (Ib) tel que représenté dans le schéma ci-dessous, avec R1, R2, R3 tels que définis précédemment à l'étape i) et/ou iv) et Z=CR4(OR5) avec R4 représente un H ou un C1-C8 alkyle ou C2-C8 alcényle, R5 représente un H et la double liaison en 2-3 ou 3-4 étant absente dans le cas où l'étape iv) est réalisée avant l'étape iii.a). 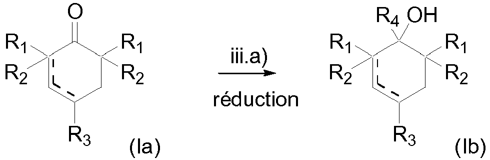 Préférentiellement, l'étape iii.a) de réduction se fait par addition d'un organomagnésien R4MgX ou d'un hydrure métallique (R4=H).

Outre l'étape iii.a), le procédé peut comprendre une étape iii.b) d'alkylation de la fonction alcool du composé (Ib) obtenu à l'étape iii.a), afin d'obtenir un composé de formule (Ic) avec R1, R2, R3 tels que définis précédemment à l'étape i) et/ou iv) et Z=CR4(OR5) avec R4 représente un H ou un C1-C8 alkyle ou C2-C8 alcényle, R5 représente un alkyle ou un alcényle (réaction de Williamson classique) et la double liaison en 2-3 ou 3-4 étant absente dans le cas où l'étape iv) est réalisée avant l'étape iii.b). Cette étape iii.b) est représentée dans le schéma ci-dessous. 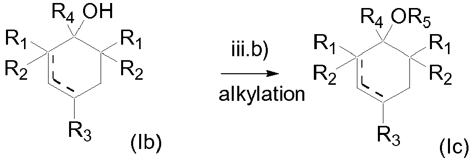 Cette étape d'alkylation permet d'obtenir des composés de formule (I) comprenant Z=CR4(OR5) avec R4 représentant un H ou un C1-C8 alkyle ou C2-C8 alcényle et R5 représentant un alkyle ou un alcényle.

Préférentiellement, l'étape d'alkylation iii.b) se fait par addition d'un halogénure d'alkyle tel que MeI, EtI, bromure d'allyle etc.

Dans un autre mode de réalisation de l'invention, l'étape iii) comprend, outre l'étape iii.a) telle que définie précédemment, une étape iii.c) d'estérification de la fonction alcool du composé (Ib) obtenu à l'étape iii.a), afin d'obtenir un composé (Id) ayant R1, R2, R3 tels que définis précédemment à l'étape i) et/ou iv) et Z=CR4(OR5) avec R4 représente un H ou un C1-C8 alkyle ou C2-C8 alcényle, et R5 représente un alcanoyle ou un alcénoyle, la double liaison en 2-3 ou 3-4 étant absente dans le cas où l'étape iv) est réalisée avant l'étape iii.c) (voir schéma ci-dessous). 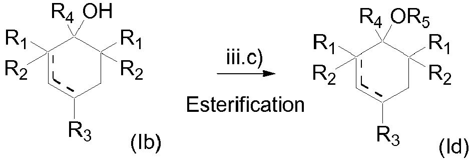 Préférentiellement, l'étape d'estérification iii.c) se fait par addition d'un anhydride ou d'un chlorure d'acyle R'C(O)Cl avec R' représentant un hydrogène, ou un alkyle en C1-C7, ou un alcényle en C2-C7.

Le procédé selon l'invention peut comprendre une étape iv) de réduction de la double liaison en 2-3 ou en 3-4 sur le cycle. Cette étape peut être réalisée après l'étape i), ii) ou iii). Elle est de préférence réalisée après l'étape i).

Dans un mode de réalisation particulier de l'invention, les composés de formule (la) avec Z=C(O) et comprenant une double liaison en 2-3 peuvent être transformés par une étape iv) de réduction sélective, en composés (Ie) où la double liaison sur le cycle est absente, la fonction Z=C(O) étant conservée (voir schéma ci-dessous). Cette étape de réduction iv) peut se faire par exemple en présence de complexes utilisant du cuivre ou du rhodium, ou selon toute autre méthode bien connue de l'homme de l'art.

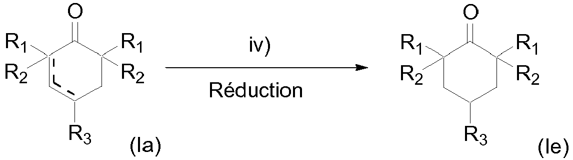

Dans un autre mode de réalisation particulier de l'invention, les composés de formule (la) ayant Z=C(O) et comprenant une double liaison en 2-3 ou en 3-4 peuvent être transformés par une étape iv) de réduction, en composés où la double liaison sur le cycle est absente et celle de la fonction Z=C(O) est conservée. Préférentiellement, cette étape de réduction iv) se fait en présence d'hydrogène et d'un catalyseur Pd/C. Dans ce cas, les composés obtenus ne portent aucune double liaison, et tous les groupements R1, R2, R3 alcényles ou alcényles cycliques éventuellement présents sont hydrogénés en groupements alkyles correspondants.

L'étape de réduction peut aussi se faire sous pression d'hydrogène en présence de Nickel de Raney afin de donner des composés de formule (I) dans lesquels la double liaison sur le cycle est absente, tous les groupements R1, R2, R3 alcényles ou alcényles cycliques éventuellement présents sont hydrogénés en groupements alkyles correspondants, et Z=CH(OH).

Ainsi, le procédé selon l'invention tel que décrit précédemment permet d'obtenir tous les composés répondant à la formule générale (I).

Un second objet de la présente invention concerne un composé de formule générale (II) suivante : 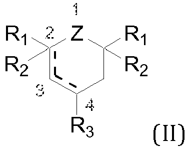 dans laquelle :
- R1 représente un méthyle ou un éthyle ;
- R2 représente indépendamment un hydrogène ou un groupement C1-C5 alkyle ou C2-C5 alcényle, branché ou linéaire ;
- R3 représente un groupement alkyle ou alcényle éventuellement substitué par un aryle, ou R3 représente un groupement alkyle cyclique comprenant cinq à six atomes de carbone ou alcényle cyclique éventuellement substitué par un ou plusieurs groupements C1-C6 alkyle, étant entendu que R3 comporte au total 7 à 10 atomes de carbone;
- Z représente C(O) ou CR4(OR5), avec
- R4 représente un hydrogène ou un groupement C1-C8 alkyle ou C2-C8 alcényle ;
- R5 représente un hydrogène ou un groupement C1-C8 alkyle ou alcanoyle, ou C2-C8 alcényle ou alcénoyle ;
sachant qu'une double liaison est présente ou absente, sur le cycle et que lorsqu'elle est présente, elle est
- soit en position 2-3 et R2 est absent en position 2,
- soit en position 3-4 et R2 est présent en position 2 et est tel que défini précédemment;
ledit composé étant sous forme d'un stéréoisomère ou d'un mélange de stéréoisomères, ou d'un mélange racémique.

Tous les composés de formule générale (II) peuvent être obtenus par l'intermédiaire du procédé décrit précédemment.

Dans un mode de réalisation particulier de l'invention, R3 est soit un groupement cyclopentyle substitué par un ou plusieurs groupements alkyles, soit un groupement cyclopentényle substitué par un ou plusieurs groupements alkyles, notamment un méthyl.

Dans un second mode de réalisation de l'invention, R3 est un groupement alkyle ou alcényle, éventuellement substitué par un aryle. Préférentiellement, R3 est substitué par un phényl.

Préférentiellement, les composés de formule (II) sont des cyclohexènones et possèdent donc un Z=C(O).

Plus préférentiellement encore, les composés de formule (II) possèdent un Z=CR4(OR5) avec R4 représente un hydrogène ou un groupement C1-C8 alkyle ou C2-C8 alcényle, et R5 représente un hydrogène.

Un troisième objet de la présente invention se rapporte à une composition comprenant au moins un composé de formule générale (II) telle que définie précédemment, sous forme d'un stéréoisomère ou d'un mélange de stéréoisomères, ou d'un mélange racémique.

Selon un mode de réalisation particulier, la composition est caractérisée en ce qu'elle comprend en outre au moins une autre substance odorante.

La quantité efficace des composés de formule (II) selon l'invention incorporée dans la composition variera selon la nature de la composition, l'effet odorant souhaité, et la nature des autres composés odorants ou non éventuellement présents, et pourra être aisément déterminée par l'homme du métier, sachant qu'elle peut varier dans une plage très étendue, de 0,1 à 99% en poids, en particulier de 0,1 à 50% en poids, notamment de 0,1 à 30% en poids par rapport au poids total de la composition.

L'invention concerne également en particulier une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, anti transpirant corporel, désodorisant corporel, lotions, crème de rasage, savon de rasage, crème, dentifrice, bain de bouche, pommade comprenant au moins un composé de formule (II), ou au moins une composition comprenant au moins un composé de formule (II).

L'invention concerne encore un produit d'entretien, notamment assouplissant, détergent, lessive, désodorisant d'ambiance, comprenant au moins un composé de formule (II) ou au moins une composition comprenant au moins un composé de formule (II).

Le ou les composés selon l'invention peuvent être utilisés, seuls ou en combinaison, tels quels ou être incorporés dans ou sur un matériau support inerte ou qui peut contenir d'autres ingrédients actifs de la composition finie. Une grande variété de matériaux supports peut être employée incluant, par exemple, les solvants polaires, les huiles, les graisses, les solides finement divisés, les cyclodextrines, les maltodextrines, les gommes, les résines et n'importe quel autre matériau support connu pour de telles compositions.

Un dernier objet de la présente invention se rapporte à l'utilisation en tant qu'agent fragrant d'un composé de formule (III) suivante : 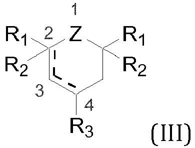 dans laquelle :
- R1 représente un méthyle ou un éthyle ;
- R2 représente indépendamment un hydrogène ou un groupement C1-C5 alkyle ou C2-C5 alcényle ;
- R3 représente alkyle ou alcényle, éventuellement substitué par un aryle, ou R3 représente un groupement alkyle cyclique comprenant cinq à six atomes de carbone ou alcényle cyclique éventuellement substitué par un ou plusieurs groupements C1-C6, étant entendu que R3 comporte au total 3 à 10 atomes de carbone et qu'il comporte au moins une insaturation lorsqu'il compte 5 à 6 atomes de carbone;
- Z représente C(O) ou CR4(OR5), avec
   - R4 représente un hydrogène ou un groupement C1-C8 alkyle ou C2-C8 alcényle ;
   - R5 représente un hydrogène ou un groupement C1-C8 alkyle ou alcanoyle, ou C2-C8 alcényle ou alcénoyle ;
   sachant qu'une double liaison est présente ou absente, sur le cycle et que lorsqu'elle est présente, elle est
   - soit en position 2-3 et R2 est absent en position 2,
   - soit en position 3-4 et R2 est présent en position 2 et est tel que défini précédemment.

Les composés de formule (III) sont utilisables comme agent masquant d'odeur ou comme agent neutralisateur d'odeur. Le terme «fragrant», est utilisé ici pour désigner tout composé organoleptique stimulant de façon plaisante l'odorat. Par le terme «agent de masquage» ou «masquant» on entend réduire ou éliminer la perception d'une mauvaise odeur générée par une ou plusieurs molécules entrant dans la composition d'un produit. Par « agent neutralisateur d'odeur » on entend rendre neutre, détruire ou absorber une mauvaise odeur fixée dans l'atmosphère ou sur un support (tel qu'un tissu). En effet, à presque toute odeur correspond une autre odeur, qui, mélangée à la première dans une certaine proportion, l'annule.

En outre, ledit composé peut être utilisé seul ou en combinaison avec au moins un autre ingrédient aromatisant ou parfumant, et/ou au moins un solvant, et/ou au moins un adjuvant. Le ou les agents odorants supplémentaires peuvent être des composés de formule (I) ou d'autres agents odorants connus de l'homme du métier qui sera à même de choisir en fonction de l'effet recherché.

De manière générale, les composés selon l'invention seront utilisés dans le domaine de la parfumerie. On entend par « parfumerie » non seulement la parfumerie au sens habituel du terme, mais également les autres domaines dans lesquels l'odeur des produits est importante. Il peut s'agir de compositions de parfumerie au sens habituel du terme, telles que bases et concentrés parfumant, eaux de Cologne, eaux de toilette, parfums et produits similaires ; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, anti-transpirants et désodorisants corporels, lotions et crèmes de rasage, savons, crèmes, dentifrices, bains de bouche, pommades, et produits similaires ; et de produits d'entretien, tels qu'assouplissants, détergents, lessives, désodorisants d'ambiance, et produits similaires.

Un mode de réalisation particulier de l'invention réside en l'utilisation d'un composé de formule (III) pour modifier ou renforcer les propriétés organoleptiques d'une substance, d'une composition ou d'un article.

On entend par «propriétés organoleptiques» toute propriété susceptible de modifier, améliorer ou renforcer la perception organoleptique d'une substance, d'une composition, d'un article par un utilisateur. Ainsi, à titre d'exemple préférentiel, l'agent organoleptique selon l'invention peut consister en un agent parfumant susceptible de conférer, modifier, améliorer ou renforcer la perception olfactive d'une substance, d'une composition ou d'un article.

Le principe général de l'invention repose sur la préparation des composés de formule (I), les nouveaux composés de formule (II), ainsi que l'utilisation en parfumerie des composés de formule (III) décrits précédemment. Les exemples suivants illustrent une manière particulière de préparer les composés de l'invention, ainsi que le profil olfactif de chacun des composés exemplifiés. Ces exemples ne sont donnés que dans un but d'illustration et ne doivent pas être compris comme limitant la portée générale de l'invention.

Le tableau suivant reprend l'ensemble des structures chimiques des composés synthétisés selon l'invention.

**Tableau 1 : Structure des composés synthétisés**

| **N°d'exemple** | **Structure chimique** | **N°d'exemple** | **Structure chimique** |
|---|---|---|---|
| **Exemple 1** | 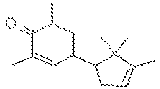 | **Exemple 24** | 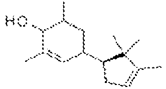 |
| **Exemple 2** | 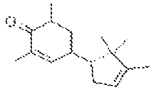 | **Exemple 25** | 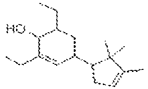 |
| **Exemple 3** | 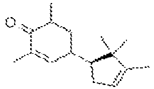 | **Exemple 26** | 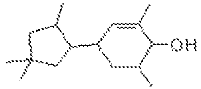 |
| **Exemple 4** | 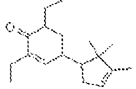 | **Exemple 27** | 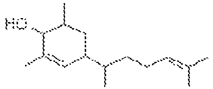 |
| **Exemple 5** | 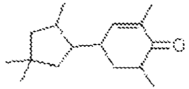 | **Exemple 28** | 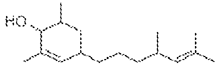 |
| **Exemple 6** | 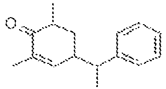 | **Exemple 29** | 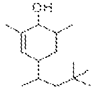 |
| **Exemple 7** | 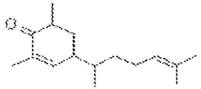 | **Exemple 30** | 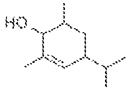 |
| **Exemple 8** | 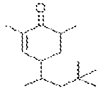 | **Exemple 31** | 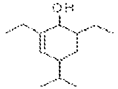 |
| **Exemple 9** | 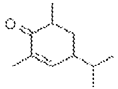 | **Exemple 32** | 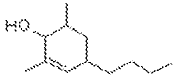 |
| **Exemple 10** | 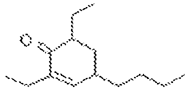 | **Exemple 33** | 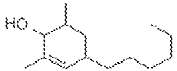 |
| **Exemple 11** | 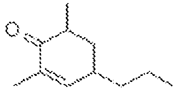 | **Exemple 34** | 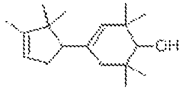 |
| **Exemple 12** | 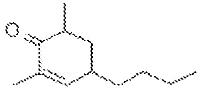 | **Exemple 35** | 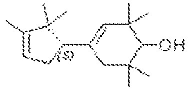 |
| **Exemple 13** | 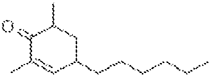 | **Exemple 36** | 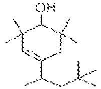 |
| **Exemple 14** | 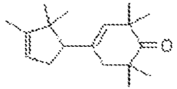 | **Exemple 37** | 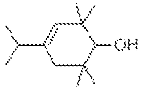 |
| **Exemple 15** | 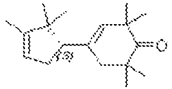 | **Exemple 38** | 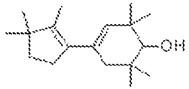 |
| **Exemple 16** | 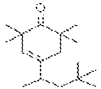 | **Exemple 39** | 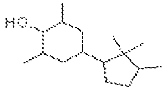 |
| **Exemple 17** | 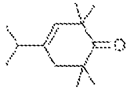 | **Exemple 40** | 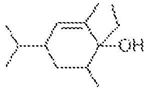 |
| **Exemple 18** | 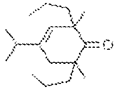 | **Exemple 41** | 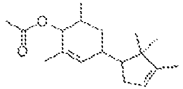 |
| **Exemple 19** | 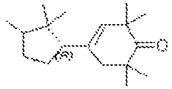 | **Exemple 42** | 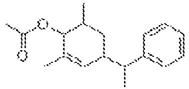 |
| **Exemple 20** | 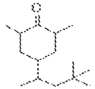 | **Exemple 43** | 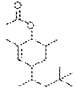 |
| **Exemple 21** | 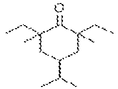 | **Exemple 44** | 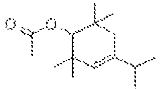 |
| **Exemple 22** | 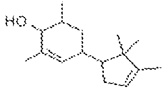 | **Exemple 45** | 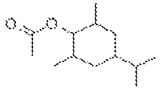 |
| **Exemple 23** | 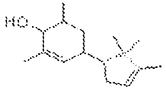 | **Exemple 46** | 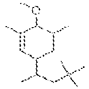 |

### Synthèse des composés exemplifiés dans le tableau 1

### Exemple 1 : Préparation de la 2,6-diméthyl-4-(2,2,3-triméthylcyclopent-3-ènyl)cyclohex-2-ènone

De la 3-pentanone (129 g, 1,5 mol, 1,5 éq.), du méthylène-campholènaldéhyde (préparé à partir de 153 g, 1 mol, 1 éq. de Campholènaldéhyde et 1.1 éq. de formaldéhyde) et de l'hydroxyde de potassium (11,2 g, 0,2 mol, 0,2 éq.) dans un mélange eau/éthanol (300 ml/200 ml) sont chauffés à 65°C pendant une nuit. Une fois la réaction terminée, le mélange réactionnel est refroidi et 0,2 éq. d'acide acétique est ajouté. La phase aqueuse est extraite 3 fois avec de l'éther de méthyle et de *t*-butyle et les phases organiques réunies sont lavées avec de la saumure, séchées sur du sulfate de magnésium et filtrées. Les solvants sont évaporés et le produit brut est purifié par distillation pour donner la 2,6-diméthyl-4-(2,2,3-triméthylcyclopent-3-ényl)cyclohex-2-énone sous forme d'une huile incolore avec un rendement de 57% sur les 2 étapes. Il s'agit d'un mélange de 4 isomères observables dans un rapport 12:40:33:15.

**Téb.** : 105-107 ° C / 0,067 kPa (0,5 torr)
**Profil olfactif** : Boisé (cèdre), ambré, épicé (poivre)
**RMN-¹H (CDCl₃, 200MHz):** δ(ppm) protons communs 1.4-1.90 (m, 2H), 1.55-1.65 (m, 3H), 1.7-1.8 (m, 3H), 1.90-2.2 (m, 2H), 2.2-2.5 (m, 2H), 2.5-2.65 (m, 1H), 5.22 (m, 1H).
Isomère majoritaire (protons caractéristiques): 0,96 (s, 3H), 1,09 (s, 3H), 1,13 (d, J = 7,04 Hz), 6,64 (s large, 1H)
Deuxième isomère majoritaire (protons caractéristiques): 0,92 (s, 3H), 1,06 (s, 3H), 1,12 (d, J = 6,67 Hz), 6,66 (s large, 1H)
Isomère minoritaire (protons caractéristiques): 0,92 (s, 3H), 1,04 (s, 3H), 1,14 (d, J = 5,68 Hz), 6,77 (s large, 1H)
Deuxième isomère minoritaire (protons caractéristiques): 0,96 (s, 3H), 1,06 (s, 3H), 1,14 (d, J = 5,68 Hz), 6,85 (br d, 1H)
**RMN-¹³C (CDCl₃, 50MHz):** δ(ppm)
Isomère majoritaire: 12,52; 15,28; 16,43 ; 19,31; 27,03 ; 34,75; 35,43; 35,62; 38,23 ; 47,31 ; 52,0 ; 121,24 ; 133,99 ; 134,94 ; 148,82 ; 202,72.
Deuxième isomère majoritaire: 12,47; 15,67; 16,43; 19,93; 27,44; 33,64; 38,54; 39,28 ; 41,57 ; 47,22 ; 53,80 ; 121,24 ; 133,99 ; 134,94 ; 148,39 ; 202,29.
Premier isomère minoritaire: 12,47; 15,67; 16,43; 20,08; 28,04; 33,95 ; 37,64; 38,85 ; 41,73 ; 46,99 ; 54,01 ; 121,19 ; 133,46 ; 134,51 ; 148,68 ; 202,32.
Deuxième isomère minoritaire: 12,52 ; 15,85 ; 16,35 ; 19,74 ; 27,24 ; 34,75 ; 35,50 ; 35,83 ; 37,69 ; 46,72 ; 53,18 ; 120,99 ; 134,51 ; 134,94 ; 148,73 ; 202,72.

### Exemple 2 : Préparation de la 2,6-diméthyl-4-((R)-2,2,3-triméthylcyclopent-3-ényl)cyclohex-2-énone

Comme décrit dans l'exemple 1, la 2,6-diméthyl-4-((R)-2,2,3-triméthylcyclopent-3-ényl)cyclohex-2-énone est préparée à partir de R-(+)-campholènaldéhyde avec un rendement de 49% sur les 2 étapes, il s'agit d'un mélange 12:36:35:17 d'isomères observables.

**Téb.** : 100°C / 0,067 kPa (0,5 torr)
**Profil olfactif** : noix, anis
Les analyses sont conformes à celles obtenues dans l'exemple 1.

### Exemple 3 : Préparation de la 2,6-diméthyl-4-((S)-2,2,3-triméthylcyclopent-3-ényl)cyclohex-2-énone

Comme décrit dans l'exemple 1, la 2,6-diméthyl-4-((S)-2,2,3-triméthylcyclopent-3-ényl)cyclohex-2-énone est préparée à partir de la S-(-)-campholenaldehyde avec un rendement de 43% sur les 2 étapes, il s'agit d'un mélange 12:43:30:15 d'isomères observables.

**Téb.** : 101-102 ° C / 0,067 kPa (0,5 torr)
**Profil olfactif** : Racines, boisé, vétiver, poivre
Les analyses sont conformes à celles obtenues dans l'exemple 1.

### Exemple 4 : Préparation de la 2,6-diéthyl-4-(2,2,3-triméthylcyclopent-3-ényl)cyclohex-2-énone

La 2,6-diéthyl-4-(2,2,3-triméthylcyclopent-3-ényl)cyclohex-2-énone est obtenue avec un rendement de 33%, selon l'exemple 1, à partir de 5-heptanone et de méthylène-campholènaldéhyde.

Il s'agit d'un mélange 32:40:28 de 3 isomères observables avec une colonne CPG apolaire.

**Téb.** : 120 ° C / 0,067 kPa (0,5 torr)
**Profil olfactif** : Epicé, curry
**RMN-¹H (CDCl₃, 200MHz):** δ(ppm) protons communs 0.8-1.2 (m, 12H), 1.3-1.6 (m, 2H), 1.6 (m, 3H), 1.6-2.1 (m, 4H), 2.15 (q, *J* = 7.4 Hz, 2H), 2.25-2.4 (m, 2H), 2.5-2.65 (m, 1H), 5.21 (m, 1H).
Isomères majoritaires (protons caractéristiques - 70%): 6.57 (br s, 1H)
Isomère minoritaire (protons caractéristiques - 15%): 6.72 (br s, 1H)
Isomère minoritaire (protons caractéristiques - 15%): 6.76 (br d, 1H)
**RMN-¹³C (CDCl₃, 50MHz):** δ(ppm)
2 Isomères majoritaires (70% 50:50): 11.23 & 11.75 (CH₃), 12.43 & 12.49 (CH₃), 13.01 & 13.09 (CH₃), 19.44 & & 20.04 (CH₃), 22.20 & 22.79 & 22.94 (2CH₂), 27.10 & 27.52 (CH₃), 32.48 & 33.48 (CH₂), 34.54 & 35.52 (CH₂), 34.92 & 38.37 (CH), 45.74 & 48.04 (CH), 47.27 (C^{IV}), 52.67 & 53.98 (CH), 121.25 & 121.31 (CH), 139.67 & 140.85 (C^{IV}), 146.44 & 148.73 (CH), 148.34 & 148.36 (C^{IV}), 201.18 & 201.25 (C(O)).
2 isomères minoritaires (30% 50:50, pics spécifiques): 11.23 & 11.57 (CH₃), 12.43 & 12.49 (CH₃), 12.83 & 12.88 (CH₃), 13.01 & 13.09 (CH₃), 19.85 & & 20.13 (CH₃), 22.23 & 22.61 & 22.71 & 22.79 (2CH₂), 27.38 & 28.03 (CH₃), 32.21 & 33.48 (CH₂), 33.97 & 34.03 (CH₂), 38.83 (CH), 45.02 & 48.18 (CH), 47.02 (C^{IV}), 53.48 & 54.30 (CH), 121.22 & 121.03 (CH), 146.58 & 146.67 (CH).

### Exemple 5 : Préparation de la 2,6-diméthyl-4-(2,4,4-triméthylcyclopentyl)cyclohex-2-énone

La 2,6-diméthyl-4-(2,4,4-triméthylcyclopentyl)cyclohex-2-énone est obtenu avec un rendement de 25%, selon l'exemple 1, à partir de la 3-pentanone et de 2-(2,4,4-triméthylcyclopentyl)acrylaldéhyde (préparé à partir de 2,4,4-triméthylcyclopentanone).

il s'agit d'un mélange de 5 isomères observables dans un rapport 16:31:34:9:5:5.
**Téb.** : 72°C / 0.040 kPa (0.3 torr)
**Profil olfactif:** Boisé sec, épicé, noix.
**RMN-¹H (CDCl₃, 200MHz):** 4 diastéréoisomères observés
δ(ppm) protons communs 0.8-1.4 (m, 15H), 1.4-1.75 (m, 3H), 1.75 (m, 3H), 1.75-2.10 (m, 2H), 2.2-2.4 (m, 1H), 2.4-2. 7 (m, 1H).
Isomères majoritaires (protons caractéristiques - 62%): 6.58 (m, 1H)
1ers isomères minoritaires (protons caractéristiques - 18%): 6.49 (m, 1H)
2èmes isomères minoritaires (protons caractéristiques - 15%): 6.59 (m, 1H)
3èmes isomères minoritaires (protons caractéristiques - 5%): 6.67 (m, 1H)
**RMN-¹³C (CDCl₃, 50MHz):** 6-7 diastéréoisomères observés
Isomères majoritaires (pics caractéristiques- 41%): 202.59 (C(O)), 147.66 & 147.35 (CH), 135.33 & 134.24 (C^{IV})
2èmes Isomères majoritaires (pics caractéristiques - 21%): 203.15 (C(O)), 149.71 (CH), 134.54 (C^{IV})
1ers isomères minoritaires (pics caractéristiques - 16%): 203.09 (C(O)), 149.15 (CH), 133.28 (C^{IV})
2èmes isomères minoritaires (pics caractéristiques - 12%): 202.49 (C(O)), 148.87 (CH), 134.54 & 134.49 (C^{IV})
3èmes isomères minoritaires (pics caractéristiques - 6%): 202.79 (C(O)), 148.07 (CH), 133.59 (C^{IV})
4èmes isomères minoritaires (pics caractéristiques - 3%): 202.96 (C(O)), 147.98 (CH), 133.49 (C^{IV})

### Exemple 6 : Préparation de la 2,6-diméthyl-4-(1-phényl-éthyl)cyclohex-2-énone

La 2,6-diméthyl-4-(1-phényl-éthyl)cyclohex-2-énone est obtenue avec un rendement de 54% au cours des 2 étapes, selon l'exemple 1, à partir de 3-pentanone et de 2-méthylène-3-phénylbutanal (préparé à partir de 3-phénylbutanal).

Il s'agit d'un mélange de 4 isomères observables dans un rapport 17:29:23:31.

**Téb.** : 115°C/0.067 kPa (0.5 torr)
**Profil olfactif** : Floral, balsamique, miellé.
**RMN-¹H (CDCl₃, 200MHz):** 4 diastéréoisomères observés
δ(ppm) protons communs 0.95-1.15 (m, 3H), 1.22-1.35 (m, 3H), 1.25-2.10 (m, 2H), 1.62-1.80 (m, 3H), 2.15-2.90 (m, 3H), 7.12-7.35 (m, 5H).
Isomères majoritaires (protons caractéristiques - 31%): 6.65-6.70 (m, 1H)
Isomères majoritaires (protons caractéristiques - 29%): 6.75-6.80 (m, 1H)
Isomères minoritaires (protons caractéristiques - 23%): 6.37-6.42 (m, 1H)
Isomères minoritaires (protons caractéristiques - 17%): 6.27-6.35 (m, 1H)
**RMN-¹³C (CDCl₃, 75MHz):** 4 diastéréoisomères observés
Isomères majoritaires (pics caractéristiques - 31%): 202.23 (C(O)), 146.42 (CH), 135.43 (C^{IV}), 38.37, 36.88 (CH₂), 17.84, 16.35, 15.15.
2èmes Isomères majoritaires (pics caractéristiques - 29%): 202.52 (C(O)), 146.69 (CH), 134.44 (C^{IV}), 40.12, 34.33 (CH₂), 19.50, 16.47, 15.61.
Isomères minoritaires (pics caractéristiques - 23%): 147.52 (CH), 134.95 (C^{IV}), 39.40, 35.33 (CH₂), 19.44, 16.22, 15.93.
2èmes isomères minoritaires (pics caractéristiques - 17%): 147.28 (CH), 133.73 (C^{IV}), 38.90, 33.39 (CH₂), 18.04, 15.15, 15.27.

### Exemple 7 : Préparation de la 2,6-diméthyl-4-(6-méthylhept-5-én-2-yl)cyclohex-2-énone

La 2,6-diméthyl-4-(6-méthylhept-5-én-2-yl)cyclohex-2-énone est obtenue avec un rendement de 51% au cours des 2 étapes, selon l'exemple 1, à partir de 3-pentanone et de 2-méthylène-citronnellal (préparé à partir de citronnellal).

Il s'agit d'un mélange de 4 isomères observables dans un rapport 25:21:28:25.

**Téb.** : 109-110°C/0.093 kPa (0.7 Torr)
**Profil olfactif** : floral, vert, racine, citronnelle, propre
**RMN-¹H (CDCl₃, 200MHz):** 3 diastéréoisomères observés
δ(ppm) protons communs 1.11 (d, J = 6.78 Hz, 3H), 1.10-1.70 (m, 4H), 1.58 (s, 3H), 1.66 (s, 3H), 1.74 (s, 3H), 1.75-2.20 (m, 3H), 2.20-2.65 (m, 2H), 5.07 (br t, 1H), 6.45-6.60 (m, 1H).
Isomères majoritaires (protons caractéristiques - 28%): 0.91 (d, J = 6.06 Hz, 3H) Autres isomères (protons caractéristiques): 0.87 (d, J = 6.76 Hz, 3H, 2 isomers) & 0.82 (d, J = 6.86 Hz, 3H)
**RMN-¹³C (CDCl₃, 75MHz):** 3 à 4 diastéréoisomères observés
203.21 & 203.15 & 202.47 (C(O)), 149.36 & 148.89 & 148.16 & 147.86 (CH), 135.38 & 135.11 & 134.09 & 133.83 (C^{IV}), 131.54 (C^{IV}), 124.26 & 124.23 & 124.15 (CH), 42.11 & 41.66 & 41.5 & 41.43 (CH), 39.55 & 39.48 & 37.61 & 37.0 (CH), 36.37 & 36.04 & 36.0 (CH), 34.94 & 34.21 & 34.19 & 33.95 (CH₂), 33.63 & 33.03 & 32.58 & 30.87 (CH₂), 25.95 & 25.88 & 25.80 & 25.74 (CH₂), 25.65 (CH₃), 17.61 & 16.29 (CH₃), 16.74 & 16.36 (CH₃), 16.27 & 16.16 & 15.73 (CH₃), 15.97 & 15.91 & 15.32 & 15.29 (CH₃).

### Exemple 8 : Préparation de la 4-(4,4-diméthylpentan-2-yl)-2,6-diméthylcyclohex-2-énone

La 4-(4,4-diméthylpentan-2-yl)-2,6-diméthylcyclohex-2-énone est obtenue avec un rendement de 56% au cours des 2 étapes, selon l'exemple 1, à partir de 3-pentanone et de 2-méthylène-3,5,5-Triméthylhexanal (préparé à partir de 3,5,5-Triméthylhexanal).

Il s'agit d'un mélange de 4 isomères observables dans un rapport 23:17:30:30.

**Téb.** : 88-92°C/0.053 kPa (0.4 torr)
**Profil olfactif** : boisé, ambré, un peu santalé, noisette.
**RMN-¹H (CDCl₃, 200MHz):** δ(ppm) 0.75-1.37 (m, 2H), 0.87-0.95 (m, 12H), 1.12 (d, J = 7.2 Hz, 3H), 1.37-2.0 (m, 3H), 1.76 (m, 3H), 2.20-2.62 (m, 2H), 6.45-6.57 (m, 1H). **RMN-¹³C (CDCl₃, 75MHz):** données sélectionnées
196.52 (C(O)), 149.06 & 148.64 & 148.60 & 148.20 (CH), 135.75 & 135.37 & 134.33 (C^{IV}), 48.46 & 47.97 & 47.92 & 47.74 (CH₂), 34.80 & 34.17 (CH₂), 32.40 & 31.67 & 31.02 (C^{IV}), 29.89 (3 CH₃).

### Exemple 9 : Préparation de la 4-isopropyl-2,6-diméthylcyclohex-2-énone

La 4-isopropyl-2,6-diméthylcyclohex-2-énone est obtenue avec un rendement de 54% au cours des 2 étapes, selon l'exemple 1, à partir de 3-pentanone et de 2-méthylène-isovaléraldéhyde (préparé à partir d'isovaléraldéhyde).

Il s'agit d'un mélange de 2 isomères observables dans un rapport 27:76.

**Téb.** : 62°C/0.133 kPa (1 torr)
**Profil olfactif** : vert, citrus, écorce de pamplemousse.
**RMN-¹H (CDCl₃, 200MHz):** protons communs 1.30-1.55 (m, 1H), 1.62-1.82 (m, 1H), 1.76 (m, 3H), 1.82-2.0 (m, 1H), 2.20-2.41 (m, 1H).
Isomères majoritaires (protons caractéristiques): δ(ppm) 0.89 (d, *J* = 6.73 Hz, 3H), 0.92 (d, *J* = 6.66 Hz, 3H), 1.12 (d, *J* = 6.64 Hz, 3H), 2.20-2.41 (m, 1H), 6.55 (m, 1H). Isomères minoritaires: δ(ppm) 0.95 (d, *J* = 6.72 Hz, 3H), 0.95 (d, *J* = 6.72 Hz, 3H), 1.12 (d, *J* = 7.2 Hz, 3H), 2.45-2.60 (m, 1H), 6.61 (m, 1H).
**RMN-¹³C (CDCl₃, 75MHz):**
Isomères majoritaires: 202.60 (C(O)), 148.33 (CH), 135.18 (C^{IV}), 42.99 (CH), 41.50 (CH), 34.32 (CH₂), 31.81 (CH), 19.40 (CH₃), 19.01 (CH₃), 16.24 (CH₃), 15.32 (CH₃). Isomères minoritaires, pics spécifiques: 148.05 (CH), 133.89 (C^{IV}), 39.28 (CH), 39.01 (CH), 32.38 (CH₂), 31.46 (CH), 20.11 (CH₃), 20.06 (CH₃), 16.35 (CH₃), 15.94 (CH₃).

### Exemple 10 : Préparation de la 4-butyl-2,6-diéthylcyclohex-2-énone

La 4-butyl-2,6-diéthylcyclohex-2-énone est obtenue avec un rendement de 16% sur les 2 étapes, selon l'exemple 1, à partir de 4-heptanone et de 2-méthylène-hexanal (préparé à partir d'hexanal).

Il s'agit d'un mélange de 2 isomères observables dans un rapport 51:49.

**Téb.** : 115°C/0.067 kPa (0.5 torr)
**Profil olfactif** : boisé, noisette.
**RMN-¹H (CDCl₃, 300MHz):** δ(ppm) 0.85-1.0 (m, 9H), 1.1-1.55 (m, 8H), 1.58-1.78 (m, 1H), 1.84-1.98 (m, 1H), 2.0-2.3 (m, 3H), 2.32-2.44 (m, 1H), 6.42-6.47 (m, 1H).
**RMN-¹³C (CDCl₃, 75MHz):** δ(ppm) 202.27 & 201.41 (C(O)), 147.62 & 147.0 (CH), 140.12 & 138.88 (C^{IV}), 47.78 & 46.25 (CH), 36.70 & 32.76 (CH), 35.61 & 34.38 (CH₂), 34.50 & 32.44 (CH₂), 29.29 & 28.72 (CH₂), 22.85 & 22.71 & 22.69 & 22.58 & 22.50 & 22.14 (3 CH₂), 13.94 (CH₃), 12.92 & 12.86 (CH₃), 11.79 & 11.11 (CH₃).

### Exemple 11 : Préparation de la 2,6-diméthyl-4-propylcyclohex-2-énone

La 2,6-diméthyl-4-propylcyclohex-2-énone est obtenue avec un rendement de 17 % sur les 2 étapes, selon l'exemple 1, à partir de la 3-pentanone et 2-méthylène-valéraldéhyde (préparé à partir de pentanal).

Il s'agit d'un mélange de 2 isomères observables dans un rapport 54:46.

**Téb.** : 70°C/0.067 kPa (0.5 torr)
**Profil olfactif** : pamplemousse, très vert, chocolat cacao.
**RMN-¹H (CDCl₃, 200MHz):**
δ(ppm) protons communs 0.85-0.97 (m, 3H), 1.1 (d, *J* = 7Hz, 3H), 1.20-1.50 (m, 4H), 1.70-1.75 (m, 3H), 1.75-2.08 (m, 2H), 2.20-2.60(m, 2H).
Isomères majoritaires (proton caractéristique): 6.54-6.59 (m, 1H)
Isomères minoritaires (proton caractéristique): 6.48-6.54 (m, 1H)
**RMN-¹³C (CDCl₃, 50MHz):**
Isomères majoritaires : 202.98 (C(O)), 148.92 (CH), 133.30 (C^{IV}), 38.52, 38.28 (CH₂), 35.58 (CH₂), 32.99, 20.53 (CH₂), 16.23, 15.72, 14.08.
Isomères minoritaires : 202.56 (C(O)), 149.56 (CH), 134.32 (C^{IV}), 41.41, 37.92 (CH₂), 36.57, 36.20 (CH₂), 19.64 (CH₂), 16.12, 15.22, 14.04.

### Exemple 12 : Préparation de la 4-butyl-2,6-diméthylcyclohex-2-ènone

La 4-butyl-2,6-diméthylcyclohex-2-ènone est obtenue avec un rendement de 40% sur les 2 étapes, selon l'exemple 1, à partir de la 3-pentanone et 2-méthylène-hexanal (préparé à partir d'hexanal).

Il s'agit d'un mélange de 2 isomères observables dans un rapport 57:43.

Téb. : 65°C/0.107 kPa (0.8 torr)
**Profil olfactif** : Vert, rhubarbe, puissant, un peu lavandé, champignon.
**RMN-¹H (CDCl₃, 200MHz):**
δ(ppm) protons communs 0.85-0.95 (m, 3H), 1.1 (d, *J* = 7Hz, 3H), 1.18-1.62 (m, 6H), 1.70-1.75 (m, 3H), 1.76-2.08 (m, 2H), 2.20-2.60(m, 2H).
Isomères majoritaires (proton caractéristique): 6.54-6.59 (m, 1H)
Isomères minoritaires (proton caractéristique): 6.48-6.54 (m, 1H)
**RMN-¹³C (CDCl₃, 50MHz):**
Isomères majoritaires : 202.93 (C(O)), 148.91 (CH), 133.27 (C^{IV}), 38.51, 35.61 (CH₂), 33.72 (CH₂), 33.23, 29.60 (CH₂), 22.72 (CH₂), 16.20, 15.71, 13.94.

Isomères minoritaires : 202.50 (C(O)), 149.56 (CH), 134.31 (C^{IV}), 41.40, 38.30 (CH₂), 36.79, 35.40 (CH₂), 28.69 (CH₂), 22.67 (CH₂), 16.10, 15.20, 13.94.

### Example 13 : Préparation of 4-hexyl-2,6-diméthylcyclohex-2-ènone

La 4-hexyl-2,6-diméthylcyclohex-2-ènone est obtenue avec un rendement de 35% sur les 2 étapes, selon l'exemple 1, à partir de la 3-pentanone et de 2-méthylène-octanal (préparé à partir d'octanal).

Il s'agit d'un mélange de 2 isomères observables dans un rapport 48:52.

**Téb.** : 105°C/ 0.067 kPa (0.5 torr)
**Profil olfactif** : aldéhydé, linge frais et propre, savon de Marseille, herbe séchée.

**RMN-¹H (CDCl₃, 200MHz):**
δ(ppm) protons communs 0.83-1.04 (m, 3H), 1.12 (d, *J* = 6.8Hz, 3H), 1.22-1.50 (m, 10H), 1.73-1.78 (m, 3H), 1.75-2.10 (m, 2H), 2.10-2.65(m, 2H).
Isomères minoritaires (proton caractéristique): 6.54-6.59 (m, 1H)
Isomères majoritaires (proton caractéristique): 6.50-6.55 (m, 1H)
**RMN-¹³C (CDCl₃, 50MHz):**

Isomères minoritaires : 198.20 (C(O)), 148.95 (CH), 38.59, 38.36 (CH₂), 35.68 (CH₂), 33.31, 31.78 (CH₂), 29.38 (CH₂), 27.44 (CH₂), 22.62 (CH₂), 16.27, 15.78, 14.06.
Isomères majoritaires : 196.54 (C(O)), 149.60 (CH), 134.38 (C^{IV}), 41.47, 36.88, 35.78 (CH₂), 34.10 (CH₂), 31.78 (CH₂), 29.33 (CH₂), 26.52 (CH₂), 22.62 (CH₂), 16.17, 15.29, 14.06.

### Exemple 14 : Préparation de la (R)-2,2,6,6-tétraméthyl-4-(2,2,3-triméthylcyclopent-3-ényl)cyclohex-3-ènone

À une solution dans le THF de 2,6-diméthyl-4-((R)-2,2,3-triméthylcyclopent-3-ényl)cyclohex-2-énone (obtenue selon l'exemple 2) sont ajoutés 1,1 équivalent molaire de t-butylate de potassium. Après 2 heures d'agitation à température ambiante, 1,1 équivalent molaire d'iodure de méthyle sont ajoutés au goutte à goutte au mélange réactionnel. 1,1 équivalent molaire de t-butylate de potassium sont encore ensuite ajoutés et le mélange réactionnel est chauffé à 40°C pendant 2 heures, puis un nouvel équivalent 1,1 molaire d'iodure de méthyle est ajouté. Après agitation à 40°C pendant une nuit, le mélange réactionnel est dilué avec de l'éther de méthyle et de t-butyle et versé dans une solution de HCl aqueux à 10%. La phase aqueuse est extraite deux fois avec de l'éther de méthyle et de t-butyle et les phases organiques réunies sont lavées avec une solution aqueuse saturée de bicarbonate de sodium. La phase organique est séchée sur sulfate de magnésium, filtrée et les solvants sont évaporés. Le produit brut ainsi obtenu est purifié par distillation pour donner la (R)-2,2,6,6-tétraméthyl-4-(2,2,3-triméthylcyclopent-3-ényl)cyclohex-3-ènone sous forme d'une huile incolore avec un rendement de 64%.

**Téb.** : 93°C/0.067 kPa (0.5 torr)
**Profil olfactif** : Poussiéreux, moisi
**IR (film, cm⁻¹):** 564m, 581m, 797m, 857w, 997w, 1013w, 1047m, 1360m, 1381m, 1466m, 1706s, 2866w, 2927m, 2958m.
**RMN-¹H (CDCl₃, 200MHz):** δ(ppm) 0.75 (s, 3H), 1.05 (s, 3H), 1.09 (s, 3H), 1.11 (s, 3H), 1.11 (s, 3H), 1.13 (s, 3H), 1.58 (m, 3H), 2.10-2.40 (m, 2H), 2.22 (q, *J* = 16.54 Hz, 2H), 2.50 (t, J = 8.22 Hz, 1H), 5.22-5.29 (m, 1H), 5.39-5.43 (m, 1H).
**RMN-¹³C (CDCl₃, 75MHz):** δ(ppm) 12.67 (CH₃), 21.10 (CH₃), 25.40 (CH₃), 25.61 (CH₃), 26.77 (CH₃), 27.19 (CH₃), 27.21 (CH₃), 33.01 (CH₂), 41.96 (CH₂), 43.08 (C^{IV}), 43.76 (C^{IV}), 48.17 (C^{IV}), 57.83 (CH), 121.36 (CH), 131.70 (CH), 134.91 (C^{IV}), 147.43 (C^{IV}), 219.92 (C(O)).

### Exemple 15 : Préparation de la (S)-2,2,6,6-tétraméthyl-4-(2,2,3-triméthylcyclopent-3-ényl)cyclohex-3-ènone

La (S)-2,2,6,6-tétraméthyl-4-(2,2,3-triméthylcyclopent-3-ényl)cyclohex-3-énone est obtenue avec un rendement de 46% selon l'exemple 14 à partir de 2,6-diméthyl-4-((S)-2,2,3-triméthylcyclopent-3-ényl)cyclohex-2-énone (obtenu dans l'exemple 3).

**Téb.** : 92°C/0.067 kPa (0.5 torr)
**Profil olfactif:** Boisé, frais, moisi
**RMN-¹H (CDCl₃, 200MHz):** δ(ppm) 0.76 (s, 3H), 1.05 (s, 3H), 1.10 (s, 3H), 1.12 (s, 6H), 1.14 (s, 3H), 1.59 (m, 3H), 2.10-2.40 (m, 2H), 2.22 (q, *J* = 16.53 Hz, 2H), 2.51 (t, J = 8.23 Hz, 1H), 5.23-5.30 (m, 1H), 5.40-5.44 (m, 1H).
**RMN-¹³C (CDCl₃, 50MHz):** δ(ppm) 12.66 (CH₃), 21.08 (CH₃), 25.39 (CH₃), 25.60 (CH₃), 26.75 (CH₃), 27.17 (CH₃), 27.19 (CH₃), 33.00 (CH₂), 41.94 (CH₂), 43.06 (C^{IV}), 43.74 (C^{IV}), 48.16 (C^{IV}), 57.81 (CH), 121.35 (CH), 131.68 (CH), 134.89 (C^{IV}), 147.41 (C^{IV}), 219.90 (C(O)).

### Exemple 16 : Préparation de la 4-(4,4-diméthylpentan-2-yl)-2,2,6,6-tetraméthylcyclohex-3-ènone

La 4-(4,4-diméthylpentan-2-yl)-2,2,6,6-tetraméthylcyclohex-3-ènone est obtenue avec un rendement de 30% selon l'exemple 14 à partir de la 4-(4,4-diméthylpentan-2-yl)-2,6-diméthylcyclohex-2-énone (obtenue dans l'exemple 9).

**Téb.** : 75°C/0.053 kPa (0.4 torr)
**Profil olfactif** : Boisé, poivré, un peu fruité et ambré.
**RMN-¹H (CDCl₃, 300MHz):** δ(ppm) 0.89 (s, 9H), 0.9-1.35 (m, 1H), 1.01 (d, *J* = 6.93 Hz, 3 H), 1.07 (s, 3H), 1.08 (s, 3H), 1.10 (s, 3H), 1.12 (s, 3H), 1.36-1.46 (m, 1H), 2.04-2.2 (m, 2H), 2.23-2.38 (m, 1H), 5.30 (s, 1H).
**RMN-¹³C (CDCl₃, 75MHz):** δ(ppm) 220.10 (C(O)), 140.23 (C^{IV}), 128.24 (CH), 48.04 (CH₂), 43.44 (C^{IV}), 42.84 (C^{IV}), 38.16 (CH₂), 37.18 (CH), 31.22 (C^{IV}), 29.88 (3 CH₃), 27.24 (CH₃), 26.46 (CH₃), 25.52, 25.43, 22.43.

### Exemple 17 : Préparation de la 4-isopropyl-2,2,6,6-tetramethylcyclohex-3-enone

La 4-isopropyl-2,2,6,6-tetramethylcyclohex-3-enone est obtenue avec un rendement de 46% selon l'exemple 14 à partir de la 4-isopropyl-2,6-diméthylcyclohex-2-énone (obtenue dans l'exemple 10).

**Téb.** : 86-87°C/1.200 kPa (9 torr)
**Profil olfactif:** camphré, terreux, boisé, animal.
**RMN-¹H (CDCl₃, 200MHz):** δ(ppm) 1.01 (d, *J* =6.81 Hz, 1H), 1.09-1.12 (m, 12H), 2.14 (s, 2H), 2.25 (hept, *J* = 13.80 Hz, 1H), 5.28 (s, 1H).
**RMN-¹³C (CDCl₃, 50MHz):** δ(ppm) 215.8 (C(O)), 140.12 (C^{IV}), 127.13 (CH), 43.41 (C^{IV}), 42.89 (C^{IV}), 39.11 (CH₂), 34.72 (CH), 27.16 (2CH₃), 25.42 (2CH₃), 20.90 (2CH₃).

### Exemple 18 : Préparation de la 4-isopropyl-2,6-diméthyl-2,6-dipropylcyclohex-3-ènone

La 4-isopropyl-2,6-diméthyl-2,6-dipropylcyclohex-3-ènone est obtenue avec un rendement de 39% selon l'exemple 14 à partir de 4-isopropyl-2,6-diméthylcyclohex-2-énone (obtenue dans l'exemple 10) et de 1-bromopropane.

**Téb.** : 75°C/0.067 kPa (0.5 torr)
**Profil olfactif** : Tête faible, transpiration, poussiéreux, boisé sec, un peu cassis. **RMN-¹H (CDCl₃, 200MHz):** δ(ppm) 0.83 (t, *J* = 6.81 Hz, 6H), 0.97-1.07 (m, 12H), 1.07-1.37 (m, 5H), 1.37-1.55 (m, 2H), 1.65-2.15 (m, 3H), 2.28 (hept, *J* = 13.70 Hz, 1H), 5.20 (s, 1H).
**RMN-¹³C (CDCl₃, 50MHz):** δ(ppm) 217.8 (C(O)), 141.33 (C^{IV}), 125.40 (CH), 47.61 (C^{IV}), 46.43 (C^{IV}), 43.86 (CH₂), 40.0 (CH₂), 37.20 (CH₂), 35.08 (CH), 26.81 (CH₃), 22.55 (CH₃), 21.14 (CH₃), 21.03 (CH₃), 18.52 (CH₂), 17.31 (CH₂), 14.60 (CH₃), 14.53 (CH₃).

### Exemple 19 : Préparation de la 2,2,6,6-tétraméthyl-4-((1R)-2,2,3-triméthylcyclo-pentyl)cyclohex-3-ènone

Une solution 1M dans le toluène de (S)-2,2,6,6-tétraméthyl-4-(2,2,3-triméthylcyclopent-3-ényl)cyclohex-3-ènone (obtenue dans l'exemple 15) avec 5% en poids de palladium 5% sur charbon est hydrogénée (p(H₂) = 20 bars) à température ambiante. Une fois la réaction terminée (suivi CPG), le mélange est filtré sur un gâteau de Célite® et les solvants sont évaporés. Le pétrole brut ainsi obtenu est distillé pour donner la 2,2,6,6-tétraméthyl-4-((1R)-2,2,3-triméthylcyclopentyl)cyclohex-3-énone et la 2,2,6,6-tétraméthyl-4-(2,3,3-triméthylcyclopent-1-ènyl)cyclohexanone (72:28) avec un rendement de 78%.

**Téb.** : 95°C/0.080 kPa (0.6 torr)
**Profil olfactif** : Animal, crésol, phénol
**2,2,6,6-tétraméthyl-4-((1R)**-**2,2,3 triméthylcyclopentyl)cyclohex-3-ènone**
**RMN-¹H (CDCl₃, 300MHz):** δ(ppm)
0.54 (s, 3H), 0.83 (d, *J* = 6.78 Hz, 3H), 0.92 (s, 3H), 1.09 (s, 3H), 1.11 (s, 6H), 1.13 (s, 3H), 1.15-1.30 (m, 1H), 1.50-1.85 (m, 4H), 2.07-2.22 (m, 1H), 2.30 (d, *J* = 15.97 Hz, 1H), 2.12 (d, *J* = 16.45 Hz, 1H), 5.35 (m, 1H).
**RMN-¹³C (CDCl₃, 75MHz):** δ(ppm) 220.14 (C(CO)), 134.45 (C^{IV}), 131.87 (CH), 58.07 (CH), 45.17 (CH), 43.79 (2 C^{IV}), 43.25 (C^{IV}), 42.14 (CH₂), 29.72 (CH₂), 27.25 (CH₃), 27.18 (CH₃), 26.60 (CH₃), 25.57 (CH₃), 25.46 (CH₂), 25.38 (CH₃), 15.68 (CH₃), 14.11 (CH₃).
**2,2,6,6-Tétraméthyl-4-(2,3,3-triméthylcyclopent-1-ènyl)cyclohexanone**
**RMN-¹H (CDCl₃, 300MHz):** δ(ppm)
0.88 (d, *J* = 6.69 Hz, 3H), 0.90 (s, 3H), 1.06 (s, 3H), 1.08 (s, 3H), 1.09 (s, 3H), 1.11 (s, 3H), 1.19 (s, 3H), 1.55-1.85 (m, 2H), 2.07-2.29 (m, 2H), 2.22 (d, *J* = 16.67 Hz, 1H), 2.34 (d, *J* = 16.02 Hz, 1H), 2.58-2.64 (m, 2H), 5.35 (m, 1H).
**RMN-¹³C (CDCl₃, 75MHz):** δ(ppm) 220.55 (C(CO)), 146.79 (C^{IV}), 122.07 (C^{IV}), 46.91 (CH), 44.16 (C^{IV}), 43.91 (C^{IV}), 43.49 (C^{IV}), 42.90 (CH₂), 39.82 (CH₂), 30.77 (CH₂), 30.67 (CH₂), 27.09 (CH₃), 26.99 (CH₃), 26.91 (CH₃), 26.85 (CH₃), 26.77 (CH₃), 20.53 (CH₃), 13.48 (CH₃).

### Exemple 20 : Préparation de la 4-(4,4-diméthylpentan-2-yl)-2,6-diméthylcyclohexanone

La 4-(4,4-diméthylpentan-2-yl)-2,6-diméthylcyclohexanone est obtenue avec un rendement de 81% selon l'exemple 19 à partir de la 4-(4,4-diméthylpentan-2-yl)-2,6-diméthylcyclohex-2-énone (obtenue dans l'exemple 9).

Il s'agit d'un mélange de 2 isomères principaux (91%) dans un rapport 29:71.

**Téb.=** 76°C/ 0.053 kPa (0.4 torr)
**Profil olfactif** : Boisé, ambré, poussiéreux, un peu fleuri.
**RMN-¹H (CDCl₃, 300MHz):** protons communs δ(ppm) 0.85-0.94 (m, 12H), 0.94-1.03 (m, 6H), 1.04-1.41 (m, 4H), 1.41-1.55 (m, 1H), 1.6-2.15 (m, 3H).
Isomères majoritaires (pic caractéristique): 2.33-2.48 (m, 2H).
Isomères minoritaires (pic caractéristique): 2.48-2.62 (m, 2H).
**RMN-¹³C (CDCl₃, 75MHz):**
Isomères majoritaires : δ(ppm) 214.81 (C(CO)), 48.54 (CH₂), 44.41 (CH), 44.30 (CH), 43.70 (CH), 40.13 (CH₂), 38.66 (CH₂), 32.76 (CH), 30.95 (C^{IV}), 29.84 (3 CH₃), 19.09 (CH₃), 14.63 (CH₃), 14.59 (CH₃).

### Exemple 21 : Préparation de la 2,6-diéthyl-4-isopropyl-2,6-diméthylcyclohexanone

La 2,6-diéthyl-4-isopropyl-2,6-diméthylcyclohexanone est obtenue avec un rendement de 40% sur 2 étapes (hydrogénation selon l'exemple 19, suivie d'une alkylation avec le bromoéthane selon l'exemple 14), à partir de la cyclohexènone obtenue dans l'exemple 10.

Il s'agit d'un mélange de stéréoisomères observables dans un rapport 6:16:57:21.

**Téb.=** 63-65°C/ 0.053 kPa (0.4 torr)
**Profil olfactif** : Boisé, un peu noix, noisette, plastique, un peu rosé.
**RMN-¹H (CDCl₃, 300MHz):** protons communs δ(ppm) 0.71-0.83 (m, 6H), 0.84-0.94 (m, 6H), 1.11-1.3 (m, 2H), 1.3-1.58 (m, 4H), 1.58-1.95 (m, 4H).
Isomères majoritaires (pic caractéristique): 0.97 (s, 6H).
Isomères minoritaires (pic caractéristique): 0.96 & 1.06 & 1.07 (s, 3H).
**RMN-¹³C (CDCl₃, 75MHz):** 4 stéréoisomères observés dont 3 majoritaires
Isomères majoritaires : δ(ppm) 220.09 & 219.40 (C(CO)), 41.45 & 40.70 & 39.31 (CH₂), 39.15 (C^{IV}), 34.18 (2C) & 34.07 (CH), 33.16 & 32.17 & 31.25 (CH₂), 32.35 (2C) & 32.21 (CH), 26.37 & 24.65 & 24.41 (CH₃), 19.84 & 19.72 & 19.62 (CH₃), 8.75 & 8.38 & 8.27 (CH₃).
Isomères minoritaires (pics caractéristiques): δ(ppm) 38.62 (CH₂), 34.28 (CH), 33.08 (CH₂), 32.27 (CH), 27.57 (CH₃), 19.72 (CH₃), 8.71 (CH₃).

### Exemple 22 : Préparation du 2,6-diméthyl-4-(2,2,3-triméthylcyclopent-3-ényl)cyclohex-2-énol

A une suspension d'hydrure de lithium et d'aluminium (5,8 g, 0,151 mol, 1,3 éq. H-) dans 500 ml d'éther diéthylique, est ajouté au goutte à goutte à 20-25°C de la 2,6-diméthyl-4-(2,2,3-triméthylcyclopent-3-ényl)cyclohex-2-énone (108 g, 0,465 mol, 1 éq., obtenue dans l'exemple 1). Une fois la réaction terminée, une solution de HCl aqueux à 10% est ajoutée goutte à goutte pour précipiter l'alumine.

Après filtration et séchage sur sulfate de magnésium, les solvants sont évaporés et le produit brut est purifié par distillation pour donner le 2,6-diméthyl-4-(2,2,3-triméthylcyclopent-3-ényl) cyclohex-2-énol sous forme d'une huile incolore avec un rendement de 76%. Elle consiste en un mélange de 5 isomères principaux observables dans un rapport 6:24:23:32:15 avec une colonne CPG apolaire.

**Téb.** : 108-110°C / 0.061 kPa (0.46 torr)
**Profil olfactif** : Santal, doux, laiteux
**RMN-¹H (CDCl₃, 200MHz):** δ(ppm) protons communs 0.88-0.93 (m, 3H), 0.93-1.15 (m, 6H), 1.42-1.72 (m, 6H), 1.72-1.81 (m, 3H), 1.81-2.07 (m, 2H), 2.11-2.4 (m, 2H), 5.21 (m, 1H).
Isomères majoritaires (protons caractéristiques, 32%): 3.65 (m, 1H), 5.43 (m, 1H) 2^{èmes} Isomères majoritaires (protons caractéristiques, 2x 20%): 3.55 (d, *J* = 4.59 Hz, 1H) & 3.78 (d, *J* = 3.57 Hz, 1H), 5.47-5.52 (m, 1H).
Isomères minoritaires (protons caractéristiques, 15%): 3.64 (m, 1H), 5.63 (m, 1H) Autres isomères minoritaires (protons caractéristiques, 6% & 7%): 3.72 & 3.90 (m, 1H), 5.66-5.74 (m, 1H)
**RMN-¹³C (CDCl₃, 75MHz):** δ(ppm)
Isomères majoritaires (32%): 148.47 (C^{IV}), 136.13 (C^{IV}), 128.59 (CH), 121.42 (CH), 76.75 (CHOH), 54.76 (CH), 47.10 (C^{IV}), 38.37 (CH), 38.09 (CH2), 37.72 (CH), 33.76 (CH₂), 19.71 (CH₃), 19.51 (CH₃), 19.37 (CH₃), 19.26 (CH₃), 12.48 (CH₃).
Isomères minoritaires (pics caractéristiques, 55%): 148.54 & 148.48 & 148.42 (C^{IV}), 135.79 & 134.97 & 134.14 (C^{IV}), 129.17 & 129.08 & 128.94 (CH), 121.42 (2C) & 121.25 (CH), 76.75 & 74.79 & 71.61 (CHOH), 54.97 & 53.71 & 52.55 (CH), 47.14 & 47.08 & 46.90 (C^{IV}).
Autres isomères minoritaires (pics caractéristiques, 6% & 7%): 149.11 & 148.89 (C^{IV}), 134.56 & 133.98 (C^{IV}), 129.13 & 129.06 (CH), 121.10 & 121.0 (CH), 75.28 & 71.43 (CHOH), 54.48 & 53.53 (CH), 46.77 & 46.62 (C^{IV}).

### Exemple 23 : Préparation du 2,6-diméthyl-4-((R)-2,2,3-triméthylcyclopent-3-ényl)cyclohex-2-énol

Le 2,6-diméthyl-4-((R)-2,2,3-triméthylcyclopent-3-ényl) cyclohex-2-énol est obtenu avec un rendement de 69%, selon l'exemple 22, à partir de la 2,6-diméthyl-4-(2,2,3-triméthylcyclopent-3-ényl)cyclohex-2-énone (obtenue dans l'exemple 2).

Il s'agit d'un mélange de 4 isomères principaux observables dans un rapport 11:15:41:33 avec une colonne CPG apolaire.

**Téb.** : 100-102°C / 0.067 kPa (0.5 torr)
**Profil olfactif** : Santal, un peu vert
Les analyses sont conformes à celles obtenues dans l'exemple 24.

### Exemple 24 : Préparation du 2,6-diméthyl-4-((S)-2,2,3-triméthylcyclopent-3-ényl)cyclohex-2-énol

Le 2,6-diméthyl-4-((S)-2,2,3-triméthylcyclopent-3-ényl)cyclohex-2-énol est obtenu avec un rendement de 66%, selon l'exemple 22, à partir de la 2,6-diméthyl-4-(2,2,3-triméthylcyclopent-3-ényl)cyclohex-2-énone (obtenue dans l'exemple 3). Il consiste en un mélange de 4 isomères principaux observables dans un rapport de 10:20:33:37 avec une colonne CPG apolaire, 7 isomères observables dans un rapport 18:6:2:15:27:24:8 avec une colonne CPG polaire GC.

**Téb.:** 100-102°C / 0.067 kPa (0.5 torr)
**Profil olfactif** : Santal, crémeux, gourmand, noisette, un peu épicé, cuiré
**IR (film, cm⁻¹):** 564w, 580w, 880m, 1046s, 1088m, 1378w, 2875w, 2971w, 3318w br.
**RMN-¹H (CDCl₃, 200MHz):** δ(ppm) protons communs 0.85-0.92 (m, 3H), 0.92-1.2 (m, 6H), 1.58 (m, 6H), 1.6-1.85 (m, 4H), 1.85-2.1 (m, 1H), 2.1-2.4 (m, 2H), 5.21 (m, 1H).
Isomère 27% (protons caractéristiques): 3.64 (d, *J* = 6.50 Hz, 1H), 5.39-5.45 (m, 1H)
Isomère 24% (protons caractéristiques): 3.55 (d, *J* = 4.83 Hz, 1H), 5.45-5.54 (m, 1H)
Isomère 18% (protons caractéristiques): 3.78 (d, *J* = 3.89 Hz, 1H), 5.45-5.54 (m, 1H)
Isomère 15% (protons caractéristiques): 3.64 (d, *J* = 6.50 Hz, 1H), 5.65-5.75 (m, 1H)
Isomère minoritaire (8%, protons caractéristiques): 3.74 (d, *J* = 3.98 Hz, 1H), 5.60-5.66 (m, 1H)
Isomère minoritaire (6%, protons caractéristiques): 3.91 (t, *J* = 6.67 Hz, 1H), 5.60-5.66 (m, 1H)
Isomère minoritaire 2% (protons caractéristiques): 3.78 (d, *J* = 3.89 Hz, 1H), 5.45-5.54 (m, 1H)
**RMN-¹³C (CDCl₃, 50MHz):** δ(ppm)
Isomères majoritaires: 12.52, 15.28, 16.43, 19.31, 27.03, 34.75, 35.43, 35.62, 38.23, 47.31, 52.0, 121.24, 133.99, 134.94, 148.82, 202.72.
2èmes Isomères majoritaires: 12.47, 15.67, 16.43, 19.93, 27.44, 33.64, 38.54, 39.28, 41.57, 47.22, 53.80, 121.24, 133.99, 134.94, 148.39, 202.29.
Isomères minoritaires: 12.47, 15.67, 16.43, 20.08, 28.04, 33.95, 37.64, 38.85, 41.73, 46.99, 54.01, 121.19, 133.46, 134.51, 148.68, 202.32.
2^{èmes} Isomères minoritaires: 12.52, 15.85, 16.35, 19.74, 27.24, 34.75, 35.50, 35.83, 37.69, 46.72, 53.18, 120.99, 134.51, 134.94, 148.73, 202.72.

### Exemple 25 : Préparation du 2,6-diéthyl-4-(2,2,3-triméthylcyclopent-3-ényl)cyclohex-2-énol

Le 2,6-diéthyl-4-(2,2,3-triméthylcyclopent-3-ényl)cyclohex-2-énol est obtenu avec un rendement de 67%, selon l'exemple 22, à partir de la 2,6-diéthyl-4-(2,2,3-triméthylcyclopent-3-ényl)cyclohex-2-énone (obtenue dans l'exemple 4).

Il s'agit d'un mélange de 4 isomères dans un rapport 4:17:32:47.

**Téb.:** 120°C / 0.060 kPa (0.45 torr)
**Profil olfactif:** Santal, plus faible que l'exemple 11
**RMN-¹H (CDCl₃, 200MHz):** δ(ppm) protons communs 0.89-0.94 (m, 3H), 0.94-1.0 (m, 3H), 1.0-1.05 (m, 3H), 1.05-1.16 (m, 3H), 1.16-1.58 (m, 4H), 1.56-1.61 (m, 3H), 1.61-2.12 (m, 4H), 2.12-2.37 (m, 3H).
Isomères 47% (protons caractéristiques): 3.85 (d, *J* = 9.32 Hz, 1H), 5.41-5.45 (m, 1H, 39%) & 5.63 (d, *J* = 4.76 Hz, 1H, 8%)
Isomères 32% (protons caractéristiques): 3.8 (d, *J* = 9.4 Hz, 1H), 5.51 & 5.63 & 5.67 (m, 1H, 11% & 14% & 7%)
Isomères 17% (protons caractéristiques): 3.65-3.73 (m, 1H), 5.48 (m, 1H)
Isomères 4% (protons caractéristiques): 3.8 (d, *J* = 9.4 Hz, 1H), 5.48 (m, 1H)
**RMN-¹³C (CDCl₃, 50MHz):** δ(ppm)
Isomères majoritaires: 148.39 (C^{IV}), 141.89 (C^{IV}), 126.81 (CH), 121.52 (CH), 68.61 (CHOH), 54.89 (CH), 47.20 (C^{IV}), 44.99 (CH), 37.54 (CH), 33.98 (CH₂), 33.55 (CH₂), 27.49 (CH₃), 25.75 (CH₂), 25.52 (CH₂), 19.91 (CH₃), 12.78 (CH₃), 12.47 (CH₃), 11.12 (CH₃).
2^{èmes} Isomères majoritaires (pics spécifiques): 127.95 (CH), 121.47 (CH), 73.39 (CHOH)
Isomères minoritaires (pics spécifiques): 127.55 & 127.08 (CH), 121.31 & 121.03 (CH), 73.51 (CHOH)

### Exemple 26 : Préparation du 2,6-diméthyl-4-(2,4,4-triméthylcyclopentyl)cyclohex-2-énol

Le 2,6-diméthyl-4-(2,4,4-triméthylcyclopentyl)cyclohex-2-énol est obtenu avec un rendement de 40%, selon l'exemple 22, à partir de la 2,6-diméthyl-4-(2,4,4-triméthylcyclopentyl) cyclohex-2-énone (obtenue dans l'exemple 5).

Le produit brut a été purifié par chromatographie sur colonne de silice, l'alcool attendu consiste en un mélange de 2 isomères principaux (83%) dans un rapport de 19:81.

**Profil olfactif** : Tabac, fumé
**RMN-¹H (CDCl₃, 200MHz):** 3 isomères observables (50:30:20)
δ(ppm) protons communs 0.80-0.97 (m, 6H), 0.97-1.02 (m, 6H), 1.02-1.20 (m, 4H), 1.20-1.75 (m, 8H), 1.72-1.8 (m, 3H), 1.8-2.0 (m, 1H), 2.0-2.35 (m, 1H).
1ers Isomères (protons caractéristiques): 3.55-3.67 (m, 1H), 5.40-5.46 (m, 1H).
2èmes Isomères (protons caractéristiques): 3.77-3.82 (m, 1H), 5.33-5.34 (m, 1H).
3èmes Isomères (protons caractéristiques): 3.50-3.55 (m, 1H), 5.26-5.32 (m, 1H).
**RMN-¹³C (CDCl₃, 50MHz):** 6 isomères observables
1ers Isomères (pics caractéristiques): 136.49 (C^{IV}), 127.49 (CH), 71.90 (CHOH), 51.90 (CH), 50.64 (CH₂), 44.08 (CH₂), 38.19 (CH), 37.07 (CH₂).
2èmes Isomères (pics caractéristiques): 135.45 (C^{IV}), 129.76 (CH), 77.14 (CHOH), 51.78 (CH), 50.56 (CH₂), 45.81 (CH₂), 38.71 (CH), 30.57 (CH₂).
3èmes Isomères (pics caractéristiques): 135.41 (C^{IV}), 127.10 (CH), 76.94 (CHOH), 51.69 (CH), 50.56 (CH₂), 43.52 (CH₂), 38.25 (CH), 32.54 (CH₂).

### Exemple 27 : Préparation du 2,6-diméthyl-4-(6-méthylhept-5-èn-2-yl)cyclohex-2-ènol

Le 2,6-diméthyl-4-(6-méthylhept-5-èn-2-yl)cyclohex-2-ènol est obtenu avec un rendement de 56%, selon l'exemple 22, à partir de la 2,6-diméthyl-4-(6-méthylhept-5-èn-2-yl)cyclohex-2-ènone (obtenue dans l'exemple 7).

Il s'agit d'un mélange d'isomères dont 1 principal (75%).

**Téb.:** 104°C/0.107 kPa (0.8 torr)
**Profil olfactif** : Rosé, citronellol, un peu boisé, plastique.
**RMN-¹H (CDCl₃, 200MHz):** 2 isomères observables (70:30)
δ(ppm) protons communs 0.75-0.85 (m, 3H), 0.95-1.20 (m, 1H), 1.20-1.57 (m, 5H), 1.59 (s, 3H), 1.68 (s, 3H), 1.82-2.22 (m, 3H), 5.03-5.15 (m, 1H).
1ers Isomères (protons caractéristiques): 1.08 (d, *J* = 6.38 Hz, 3H), 1.72-1.76 (m, 3H), 3.63 (d large, *J* = 8.55 Hz, 1H), 5.26-5.34 (m, 1H).
2èmes Isomères (protons caractéristiques): 0.97 (d, *J* = 6.88 Hz, 3H), 1.76-1.80 (m, 3H), 3.88 (t large, 1H), 5.36-5.44 (m, 1H).
**RMN-¹³C (CDCl₃, 50MHz):** 4 isomères observables (2 majoritaires)
δ(ppm) pics communs 131.18 (C^{IV}), 124.82 & 124.77 (CH), 25.69 (CH₃), 19.41 & 19.38 & 19.34.
1ers Isomères (pics caractéristiques): 136.39 & 136.04 (C^{IV}), 129.35 & 128.15 (CH), 77.11 & 77.06 (CHOH), 41.33 & 40.78 (CH), 38.55 & 38.41 (CH), 36.71 & 36.69 (CH), 34.02 & 33.77 (CH₂), 33.50 & 31.95 (CH₂), 26.12 & 26.02 (CH₂), 19.25 & 17.62 (CH₃), 16.24 & 15.21 (CH₃).
2èmes Isomères (pics caractéristiques): 135.41 & 135.10 (C^{IV}), 128.25 & 127.32 (CH), 71.92 (2 CHOH), 38.27 & 37.80 (CH), 36.83 & 36.59 (CH), 34.53 & 34.44 (CH₂), 31.79 & 31.73 (CH), 28.70 & 27.41 (CH₂), 25.89 (2x CH₂), 20.87 & 20.76 (CH₃), 17.09 & 16.70 (CH₃), 15.21 & 15.08 (CH₃).

### Exemple 28 : Préparation du 4-(4,6-diméthylhept-5-ènyl)-2,6-diméthylcyclohex-2-ènol

Le 4-(4,6-diméthylhept-5-ènyl)-2,6-diméthylcyclohex-2-ènol est obtenu avec un rendement de 70%, selon l'exemple 22, à partir de la 4-(4,6-diméthylhept-5-ènyl)-2,6-diméthylcyclohex-2-ènone (obtenue, selon l'exemple 1, avec un rendement de 16% au cours des 2 étapes, à partir de 3-pentanone et de 5,7-diméthyl-2-méthylène-oct-6-énal (préparé à partir de 5,7-diméthyl-oct-6-énal)).

Le produit brut est purifié par chromatographie sur colonne de silice, l'alcool attendu consiste en un mélange d'isomères dont 2 principaux (74%) dans un rapport de 32:68.

**Profil olfactif** : Tête faible, un peu savonneux, fruité, puis boisé, poussiéreux.
**RMN-¹H (CDCl₃, 300MHz):** 3 isomères observables (60:27:13)
δ(ppm) protons communs 0.89 (d, *J* = 6.60 Hz, 3H), 0.89-0.97 (m, 1H), 1.14-1.47 (m, 6H), 1.60 (s, 3H), 1.68 (s, 3H), 1.72-1.79 (m, 3H), 1.94-2.14 (m, 1H)
0.75-0.85 (m, 3H), 0.95-1.20 (m, 1H), 1.20-1.57 (m, 5H), 1.59 (s, 3H), 1.68 (s, 3H), 1.82-2.22 (m, 3H), 5.03-5.15 (m, 1H).
Isomères majoritaires (protons caractéristiques): 1.08 (d, *J* = 6.48 Hz, 3H), 2.16-2.38 (m, 2H), 3.64 (d large, *J* = 7.8 Hz, 1H), 5.32 (m, 1H).
1ers isomères minoritaires (protons caractéristiques): 1.12 (d, *J* = 6.78 Hz, 3H), 1.80-1.92 (m, 2H), 3.74 (d, *J* = 3.75 Hz, 1H), 5.45 (m, 1H).
2èmes isomères minoritaires (protons caractéristiques): 0.99 (d, *J* = 6.87 Hz, 3H), 2.38-2.61 (2H), 3.55 (d, *J* = 4.68 Hz, 1H), 5.42 (m, 1H).
**RMN-¹³C (CDCl₃, 75MHz):** 3 isomères observables (2 majoritaires)
1ers Isomères majoritaires: δ(ppm) 135.30 (C^{IV}), 130.04 & 130.0 (CH), 129.59 (C^{IV}), 76.96 (CHOH), 38.28 (CH), 37.92 & 37.85 (CH₂), 37.42 & 37.37 (CH₂), 36.84 & 36.79 (CH₂), 36.09 (CH), 32.3, 32.26, 25.75 (CH₃), 24.45 & 24.42 (CH₂), 19.28 (CH₃), 19.25 (CH₃), 17.91 (CH₃).
2èmes Isomères (pics caractéristiques): δ(ppm) 134.39 (C^{IV}), 129.52 & 129.44 (CH), 129.58 (C^{IV}), 71.77 (CHOH).

### Exemple 29 : Préparation du 4-(4,4-diméthylpentan-2-yl)-2,6-diméthylcyclohex-2-ènol

Le 4-(4,4-diméthylpentan-2-yl)-2,6-diméthylcyclohex-2-ènol est obtenu avec un rendement de 63%, selon l'exemple 22, à partir de la 4-(4,4-diméthylpentan-2-yl)-2,6-diméthylcyclohex-2-ènone (obtenue dans l'exemple 9).

Il consiste en un mélange de 6 isomères dont 3 principaux (80%) dans un rapport de 30:45:25.

**Téb.:** 90°C/0.053 kPa (0.4 torr)
**Profil olfactif** : boisé, santalé, ambré, un peu fleuri et musqué.
**RMN-¹H (CDCl₃, 200MHz):** 3 isomères observables majoritairement
δ(ppm) protons communs 0.8-1.05 (m, 12H), 1.0-1.65 (m, 5H), 1.65-2.50 (m, 2H).
Isomères majoritaires (protons caractéristiques): 1.09 (d, *J* = 6.31 Hz, 3H), 1.72-1.76 (m, 3H), 3.59-3.68 (m, 1H), 5.25-5.33 (m, 1H).
Isomères minoritaires (protons caractéristiques): 0.9-1.0 (m, 3H), 1.76-1.80 (m, 3H), 3.86-3.91 et 3.91-3.97 (2m, 1H), 5.34-5.39 et 5.39-5.44 (2m, 1H).
**RMN-¹³C (CDCl₃, 50MHz):** 4 isomères observables (2 majoritaires)
Isomères majoritaires (pics caractéristiques): 136.69 & 136.27 (C^{IV}), 129.22 & 128.62 (CH), 77.17 & 77.11 (CHOH), 47.79 & 47.64 (CH₂), 43.47 & 43.30 (CH), 38.50 & 38.47 (CH), 33.47 & 32.77 (CH₂), 33.24 & 32.91 (CH), 29.97 (3 CH₃), 28.79 & 27.91 (C^{IV}).
Isomères minoritaires (pics caractéristiques): 128.29 & 127.58 (CH), 72.08 & 71.95 (CHOH), 48.80 & 48.52 (CH₂), 40.16 & 39.49 (CH), 33.38 & 33.09 (CH), 32.11 & 31.66 (CH), 31.07 & 30.98 (CH₂), 29.97 (3 CH₃), 28.79 & 27.91 (C^{IV}).

### Exemple 30 : Préparation du 4-isopropyl-2,6-diméthylcyclohex-2-ènol

Le 4-isopropyl-2,6-dimethylcyclohex-2-enol est obtenu avec un rendement de 73%, selon l'exemple 22, à partir de la 4-isopropyl-2,6-diméthylcyclohex-2-ènone (obtenue dans l'exemple 10).

Il consiste en un mélange de 4 isomères observables dont 2 principaux (86%) dans un rapport de 20:80.

**Téb.:** 60°C/0.064 kPa (0.48 torr)
**Profil olfactif** : floral, rosé, citronellol.
**RMN-¹H (CDCl₃, 300MHz):** 3 isomères observables, 2 principaux (20:80)
δ(ppm) protons communs 1.3-1.63 (m, 3H), 1.8-2.13 (m, 2H).
Isomères majoritaires (protons caractéristiques): 0.81 (d, *J* = 6.84 Hz, 3H), 0.84 (d, *J* = 6.78 Hz, 3H), 1.07 (d, *J* = 6.33 Hz, 3H), 1.70-1.74 (m, 3H),
Isomères minoritaires (protons caractéristiques): 0.86 (d, *J* = 6.66 Hz, 3H), 0.88 (d, *J* = 6.69 Hz, 3H), 0.95 (d, *J* = 6.87 Hz, 3H), 1.75-1.78 (m, 3H)
**RMN-¹³C (CDCl₃, 75MHz):** 2 isomères observables
Isomères majoritaires: δ(ppm) 136.29 (C^{IV}), 128.21 (CH), 76.81 (CHOH), 42.27 (CH), 38.23 (CH), 33.24 (CH₂), 32.07 (CH), 19.37 (CH₃), 19.34 (CH₃), 19.31(CH₃), 18.47 (CH₃).
Isomères majoritaires: δ(ppm) 135.12 (C^{IV}), 127.63 (CH), 71.60 (CHOH), 39.86 (CH), 31.87 (CH), 31.36 (CH), 28.09 (CH₂), 20.87 (CH₃), 20.50 (CH₃), 20.47 (CH₃), 15.42 (CH₃).

### Exemple 31 : Préparation du 2,6-diéthyl-4-isopropylcyclohex-2-ènol

Le 2,6-diéthyl-4-isopropylcyclohex-2-ènol est obtenu avec un rendement de 72%, selon l'exemple 22, à partir de la 2,6-diéthyl-4-isopropylcyclohex-2-ènone (obtenue avec un rendement de 27% sur les 2 étapes, selon l'exemple 1, à partir de 4-heptanone et de 2-méthylène-isovaléraldéhyde (préparé à partir d'isovaléraldéhyde)).

Il consiste en un mélange de 4 isomères observables dont 2 principaux (83%) dans un rapport de 46:54.

**Téb.:** 72°C/0.060 kPa (0.45torr)
**Profil olfactif** : boisé, fruité.
**RMN-¹H (CDCl₃, 300MHz):** 3 isomères observables, 2 principaux (45:55)
δ(ppm) protons communs 0.81-0.98 (m, 9H), 0.98-1.07 (m, 3H), 1.12-1.43 (m, 2H), 1.43-1.63 (m, 3H), 1.63-1.75 & 1.91-2.04 (m, 1H), 1.76-1.90 (m, 1H), 2.04-2.26 (m, 1H).
Isomères majoritaires (protons caractéristiques): 3.78 (d large, *J* = 8.79 Hz, 1H), 5.35 (s, 1H).
1ers Isomères minoritaires (protons caractéristiques): 3.88 (s, 1H), 5.51 (m, 1H).
2^{èmes} Isomères minoritaires (protons caractéristiques): 3.69 (m, 1H), 5.42 (m, 1H).
**RMN-¹³C (CDCl₃, 75MHz):** 2 isomères observables
Isomères majoritaires: δ(ppm) 141.11 (C^{IV}), 126.73 (CH), 68.87 (CHOH), 42.12 (CH), 40.37 (CH), 32.34 (CH), 29.08 (CH₂), 25.69 (CH₂), 25.65 (CH₂), 20.92 (CH₃), 19.98 (CH₃), 12.87 (CH₃), 11.18 (CH₃).
2èmes Isomères majoritaires: δ(ppm) 142.02 (C^{IV}), 126.67 (CH), 73.63 (CHOH), 44.94 (CH), 38.26 (CH), 32.06 (CH), 27.57 (CH₂), 25.15 (CH₂), 23.73 (CH₂), 20.92 (CH₃), 19.93 (CH₃), 12.89 (CH₃), 11.84 (CH₃).
Isomères minoritaires (pics caractéristiques): 139.88 (C^{IV}), 126.97 (CH), 71.08 (CHOH).

### Exemple 32 : Préparation du 4-butyl-2,6-diméthylcyclohex-2-ènol

Le 4-butyl-2,6-diméthylcyclohex-2-ènol est obtenu avec un rendement de 71%, selon l'exemple 22, à partir de la 4-butyl-2,6-diméthylcyclohex-2-ènone (obtenue dans l'exemple 12).

Il consiste en un mélange de 3 isomères observables dont 2 principaux (89%) dans un rapport de 18:82.

**Téb.:** 90°C / 0.067 kPa (0.5 torr)
**Profil olfactif** : Citrus (peau de citrus confite), pamplemousse, soufré, rhubarbe. **RMN-¹H (CDCl₃, 300MHz):** 3 isomères observables (50:30:30)
δ(ppm) protons communs 0.83-0.92 (m, 3H), 1.1-1.36 (m, 6H), 1.36-1.70 (m, 2H), 1.77-1.99 (m, 1H), 1.99-2.77 (m, 1H).
Isomères majoritaires (protons caractéristiques): 1.06 (d, *J* = 6.51 Hz, 3H), 1.70-1.73 (m, 3H), 3.60 (d large, *J* = 8.4 Hz, 1H), 5.31 (m, 1H).
1ers Isomères minoritaires (protons caractéristiques): 0.97 (d, *J* = 6.87 Hz, 3H), 1.75-1.77 (m, 3H), 3.72 (d large, *J* = 2.8 Hz, 1H), 5.51 (m, 1H).
2^{èmes} Isomères minoritaires (protons caractéristiques): 0.93 (d, *J* = 6.96 Hz, 3H), 1.73-1.75 (m, 3H), 3.52 (d large, *J* = 3.9 Hz, 1H), 5.45 (d large, *J* = 4.11 Hz, 1H).
**RMN-¹³C (CDCl₃, 75MHz):** 3 isomères observables
Isomères majoritaires: δ(ppm) 135.48 (C^{IV}), 129.84 (CH), 76.82 (CHOH), 38.19 (CH), 37.39 (CH₂), 36.33 (CH₂), 36.04 (CH), 33.99 (CH₃), 28.77 (CH₂), 22.81 (CH₂), 19.26 (CH₃), 14.02 (CH₃).
2èmes Isomères majoritaires (pics caractéristiques): δ(ppm) 134.46 (C^{IV}), 129.33 (CH), 71.66 (CHOH), 34.53 (CH₂), 29.93 (CH₂), 22.84 (CH₂).
2^{èmes} Isomères minoritaires (pics caractéristiques): 133.60 (C^{IV}), 129.44 (CH), 74.72 (CHOH).

### Exemple 33 : Préparation du 4-hexyl-2,6-diméthylcyclohex-2-ènol

Le 4-hexyl-2,6-diméthylcyclohex-2-ènol est obtenu avec un rendement de 55%, selon l'exemple 22, à partir de la 4-hexyl-2,6-dimethylcyclohex-2-ènone (obtenue dans l'exemple 13).

Il consiste en un mélange de 3 isomères observables dont 2 principaux (88%) dans un rapport de 17:83.

**Téb.:** 90°C/0.061 kPa (0.46 torr)
**Profil olfactif** : alcool gras, savonneux.
**RMN-¹H (CDCl₃, 300MHz):** 3 isomères observables (50:30:30)
δ(ppm) protons communs 0.8-0.88 (m, 3H), 1.06-1.36 (m, 10H), 1.36-1.86 (m, 2H), 1.88-2.12 (m, 1H), 2.12-2.69 (m, 1H).
Isomères majoritaires (protons caractéristiques): 1.03 (d, *J* = 6.48 Hz, 3H), 1.68-1.70 (m, 3H), 3.56 (d large, *J* = 8.56 Hz, 1H), 5.28 (m, 1H).
1ers Isomères minoritaires (protons caractéristiques): 0.95 (d, *J* = 6.84 Hz, 3H), 1.73-1.75 (m, 3H), 3.68 (d, *J* = 3.78 Hz, 1H), 5.42 (d, *J* = 3.60 Hz, 1H).
2^{èmes} Isomères minoritaires (protons caractéristiques): 0.91 (d, *J* = 6.93 Hz, 3H), 1.70-1.73 (m, 3H), 3.49 (d, *J* = 4.75 Hz, 1H), 5.38 (m, 1H).
**RMN-¹³C (CDCl₃, 75MHz):** 3 isomères observables
Isomères majoritaires: δ(ppm) 135.63 (C^{IV}), 129.60 (CH), 76.64 (CHOH), 38.05 (CH), 37.42 (CH₂), 36.64 (CH₂), 36.05 (CH), 34.0 (CH₃), 31.78 (CH₂), 30.36 (CH₃), 29.45 (CH₂), 29.43 (CH₂), 22.56 (CH₂), 13.97 (CH₃).
2èmes Isomères majoritaires (pics caractéristiques): δ(ppm) 134.46 (C^{IV}), 129.16 (CH), 71.49 (CHOH).
2^{èmes} Isomères minoritaires (pics caractéristiques): 133.69 (C^{IV}), 130.49 (CH), 74.61 (CHOH).

### Exemple 34 : Préparation du 2,2,6,6-tétraméthyl-4-((R)-2,2,3-triméthylcyclopent-3-ényl)cyclohex-3-énol

Le 2,2,6,6-tétraméthyl-4-((R)-2,2,3-triméthylcyclopent-3-ényl)cyclohex-3-énol est obtenu avec un rendement de 64%, selon l'exemple 22, à partir de la (R)-2,2,6,6-tétraméthyl-4-(2,2,3-triméthylcyclopent-3-ényl)cyclohex-3-ènone (obtenu dans l'exemple 14).

Il s'agit d'un mélange de 2 isomères dans un rapport 44:56.

**Téb.:** 100°C / 0.067 kPa (0.5 torr)
**Profil olfactif:** Boisé, faible.
**RMN-¹H (CDCl₃, 200MHz,** protons communs): δ(ppm) 1.43 (d, *J* = 5.38 Hz, 1H), 1.55-1.62 (m, 3H), 1.72-2.0 (m, 2H), 2.02-2.20 (m, 1H), 2.32-2.45 (m, 1H), 3.31 (d, *J* = 5.53 Hz, 1H), 5.21-5.28 (m, 1H).
Isomères majoritaires: 0.74 (s, 3H), 0.94 (s, 3H), 1.01 (s, 6H), 1.04 (s, 3H), 1.06 (s, 3H), 2.20-2.26 (m, 1H), 5.17 (s, 1H).
Isomères minoritaires: 0.77 (s, 3H), 0.93 (s, 3H), 1.0 (s, 6H), 1.01 (s, 3H), 1.07 (s, 3H), 2.26-2.32 (m, 1H), 5.18 (s, 1H).
**RMN-¹³C (CDCl₃, 50MHz):** δ(ppm)
Isomères majoritaires: 12.74, 20.67, 20.71, 22.15, 26.70, 29.38, 31.79, 32.71, 35.38, 37.31, 44.30, 48.12, 57.55, 82.54, 121.54, 132.42, 132.81, 147.47.
Isomères minoritaires: 12.74, 20.92, 21.28, 22.33, 26.92, 29.23, 31.63, 33.57, 35.35, 37.26, 42.88, 48.08, 57.16, 82.56, 121.52, 132.98, 133.23, 148.09.

### Exemple 35 : Préparation du 2,2,6,6-tétraméthyl-4-((S)-2,2,3-triméthylcyclopent-3-ényl)cyclohex-3-énol

Le 2,2,6,6-tétraméthyl-4-((S)-2,2,3-triméthylcyclopent-3-ényl)cyclohex-3-énol est obtenu avec un rendement de 91% (97% de pureté), selon l'exemple 22, à partir de (S)-2,2,6,6-tétraméthyl-4-(2,2,3-triméthylcyclopent-3-ényl)cyclohex-3-ènone (obtenue dans l'exemple 15).

Il s'agit d'un mélange de 2 isomères dans un rapport 43:57.

**Téb.:** 100-103°C / 0.067 kPa (0.5 torr)
**Profil olfactif** : fruité, framboise.
Analyses similaires à celles de l'exemple 38.

### Exemple 36 : Préparation du 4-(4,4-diméthylpentan-2-yl)-2,2,6,6-tetraméthylcyclohex-3-ènol

Le 4-(4,4-diméthylpentan-2-yl)-2,2,6,6-tetraméthylcyclohex-3-ènol est obtenu avec un rendement de 28%, sur 2 étapes (alkylation selon l'exemple 14, suivie d'une réduction de la cétone selon l'exemple 22), à partir de la cyclohexènone obtenue dans l'exemple 9.

**Téb.:** 75°C / 0.053 kPa (0.4 torr)
**Profil olfactif:** terreux, moisi
**RMN-¹H (CDCl₃, 200MHz):** δ(ppm) 0.88 (s, 9H), 0.88-.92 (d, 3H), 0.92-0.98 (m, 3H), 1.0-1.05 (m, 3H), 1.3-1.5 (m, 2H), 1.65-1.95 (m, 2H), 2.05-2.30 (m, 1H), 3.28 (d, *J* = 2.19 Hz, 1H), 5.07 (dd, *J* = 4.97, 2.26 Hz, 1H).
**RMN-¹³C (CDCl₃, 50MHz):** δ(ppm)
138.75 & 138.41 (C^{IV}), 129.93 & 128.98 (CH), 82.79 & 82.59 (CHOH), 48.76 & 47.94 (CH₂), 40.03 & 39.22 (CH₂), 37.55 & 37.07 (CH), 37.03 & 36.96 (C^{IV}), 35.02 (C^{IV}), 31.33 & 30.98 (CH), 31.25 (C^{IV}), 30.02 & 29.88 (3 CH₃), 29.24 & 21.99 (CH₃), 22.63 (CH₃), 20.82 & 20.26 (CH₃).

### Example 37 : Préparation du 4-isopropyl-2,2,6,6-tetraméthylcyclohex-3-ènol

Le 4-isopropyl-2,2,6,6-tetraméthylcyclohex-3-ènol est obtenu avec un rendement de 47%, en traitant une solution éthanolique de 4-isopropyl-2,2,6,6-tetraméthylcyclohex-3-ènone (obtenue dans l'exemple 19), à 0°C, par du NaBH₄ (0.5 éq.). Une fois la transformation terminée (suivi CPG), l'éthanol est évaporé de moitié et le mélange dilué dans l'éther de méthyle et de t-butyle. Une solution d'HCl 34% aq. est alors ajoutée et la phase aqueuse, décantée, extraite 2 fois au MTBE. Les phases organiques réunies sont lavées avec une solution aqueuse saturée de bicarbonate de sodium, puis avec de la saumure, séchées sur sulfate de magnésium et les solvants sont évaporés. Le produit brut est purifié par distillation.

**Téb.:** 70°C / 0.053 kPa (0.4 torr)
**Profil olfactif:** boisé, terreux, camphré, transpiration
**RMN-¹H (CDCl₃, 300MHz):** δ(ppm) 0.89 (s, 3H), 0.93-0.99 (m, 9H), 1.02 (s, 3H), 1.03 (s, 3H), 1.54 (s large, 1 OH), 1.80 (dd, *J* = 45.77 Hz, *J* = 16.72 Hz, *J* = 2.37 Hz, 2H), 2.12 (hept, *J* = 6.81 Hz, 1H), 3.29 (s, 1H), 5.05 (dd, J = 2.40 Hz, J = 0.84 Hz, 1H).
**RMN-¹³C (CDCl₃, 75MHz):** δ(ppm)
138.24 (C^{IV}), 128.29 (CH), 82.61 (CHOH), 40.35 (CH₂), 36.91 (C^{IV}), 34.98 (C^{IV}), 34.71 (CH), 31.45 (CH₃), 29.16 (CH₃), 22.14 (CH₃), 21.30 (CH₃), 21.02 (CH₃), 20.44 (CH₃).

### Exemple 38 : Préparation du 2,2,6,6-tétraméthyl-4-(2,3,3-triméthylcyclopent-1-ényl)cyclohex-3-énol

Le 2,2,6,6-tétraméthyl-4-(2,3,3-triméthylcyclopent-1-ényl) cyclohex-3-énol est obtenu par traitement d'une solution 1M de 2,2,6,6-tétraméthyl-4-((S)-2,2,3-triméthylcyclopent-3-ényl)cyclohex-3-énol (obtenu dans l'exemple 25) avec de l'acide triflique à 50°C. Une fois la réaction terminée (suivi CPG), le mélange est versé sur une solution aqueuse saturée de bicarbonate de sodium. La phase aqueuse est extraite deux fois avec du toluène et les phases organiques réunies sont lavées avec de la saumure, séchées sur du sulfate de magnésium et les solvants sont évaporés. Le produit brut est distillé pour donner le 2,2,6,6-tétraméthyl-4-(2,3,3-triméthylcyclopent-1-ényl)cyclohex-3-énol avec un rendement de 53%.

**Téb.:** 100°C / 0.067 kPa (0.5 torr)
**Profil olfactif** : Poussiéreux, légumes crus, moisi
**RMN-¹H (CDCl₃, 200MHz):** δ(ppm) 1.09 (s, 3H), 1.04 (s, 6H), 1.02 (s, 3H), 0.97 (s, 3H), 0.94 (s, 3H), 1.53-1.64 (m, 2H), 1.58-1.60 (m, 3H), 1.80-2.12 (m, 2H), 2.20-2.32 (m, 2H), 3.34 (s, 1H), 5.17 (d, *J* = 2.29 Hz, 1H).
**RMN-¹³C (CDCl₃, 50MHz):** δ(ppm)
140.08 (C^{IV}), 135.11 (C^{IV}), 133.62 (CH), 130.64 (C^{IV}), 82.25 (CHOH), 47.26 (C^{IV}), 42.51 (CH₂), 38.61 (CH₂), 37.38 (C^{IV}), 35.16 (C^{IV}), 32.48 (CH₂), 31.46 (CH₃), 29.15 (CH₃), 26.27 (CH₃), 26.14 (CH₃), 22.19 (CH₃), 20.72 (CH₃), 10.93 (CH₃).

### Exemple 39 : Préparation du 2,6-diméthyl-4-(2,2,3-triméthylcyclopentyl)cyclohexanol

Le 2,6-diméthyl-4-(2,2,3-triméthylcyclopentyl)cyclohexanol est obtenu selon l'exemple 19 à partir du 2,6-diméthyl-4-(2,2,3-triméthylcyclopent-3-ényl)cyclohex-2-énol (obtenu dans l'exemple 22) à 60°C sous 30 bars de H₂.

Il s'agit d'un mélange de 8 stéréoisomères dans un rapport 6:11:12:35:7:5:12:12.

**Téb.:** 105°C / 0.067 kPa (0.5 torr)
**Profil olfactif** : Aldéhydé, gras, un peu boisé sec.
**RMN-¹H (CDCl₃, 200MHz):** δ 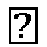 (ppm) 0.54-.69 (m, 3H), 0.7-0.85 (m, 3H), 0.85-1.04 (m, 9H), 1.04-1.8 (m, 12H), 1.8-2.12 (m, 2H), 3.19 (dd, *J =* 10.24, 4.89 Hz, 1H) et 3.38-3.54 (m, 1H).
**RMN-¹³C (CDCl₃, 50MHz):** spectre complexe correspondent à 6 stéréroisomères (Pics caractéristiques) δ(ppm) 82.87 & 81.80 (majoritaire) & 78.45 & 78.40 & 75.09 & 74.85 (CHOH).

### Exemple 40: Préparation du 1-éthyl-4-isopropyl-2,6-diméthylcyclohex-2-èn-1-ol

A une solution dans le THF de 4-isopropyl-2,6-diméthylcyclohex-2-ènone (obtenue dans l'exemple 10) est ajoutée, à 0°C, une solution 1M de chlorure d'éthylmagnésien dans le THF (1.2 éq.). Une fois la réaction terminée (suivi CPG), le milieu réactionnel est versé lentement dans un mélange éther de méthyle et de t-butyle (MTBE) / HCl 10% aq. à 0°C. La phase aqueuse est extraite 2 fois au MTBE et les phases organiques réunies sont lavées avec une solution aqueuse saturée de bicarbonate de sodium, puis à la saumure. Après séchage sur sulfate de magnésium, filtration sur papier et évaporation des solvants, le produit brut est purifié par distillation sous pression réduite pour donner le 1-éthyl-4-isopropyl-2,6-diméthylcyclohex-2-èn-1-ol avec un rendement de 66%.

Il s'agit d'un mélange de stéréoisomères dont 4 principaux (71%) dans un rapport 44:14:28:14.

**Téb.:** 67°C /0.053 kPa (0.4 torr)
**Profil olfactif** : Boisé, camphré, un peu moisi
**RMN-¹H (CDCl₃, 200MHz):** δ(ppm) 0.60-1.15 (m, 12H), 1.15-1.65 (m, 4H), 1.65-1.80 (m, 3H), 1.80-2.25 (m, 2H), 2.25-3.0 (m, 1H), 5.35 & 5.42 & 5.49 (m, 1H).
**RMN-¹³C (CDCl₃, 50MHz):** spectre complexe correspondent à 6 stéréroisomères (Pics caractéristiques) δ(ppm) 131.85 (CH, majo), 105.46 (C^{IV}, majo).

### Exemple 41: Préparation de l'acétate de 2,6-diméthyl-4-(2,2,3-triméthylcyclopent-3-ényl)cyclohex-2-ényle

Le 2,6-diméthyl-4-(2,2,3-triméthylcyclopent-3-ényl)cyclohex-2-ényle acétate est obtenu en traitant le 2,6-diméthyl-4-(2,2,3-triméthylcyclopent-3-ényl)cyclohex-2-énol (obtenu dans l'exemple 19) avec 1,2 équivalent molaire d'anhydride acétique et une quantité catalytique de N,N-diméthylaminopyridine. Après 2 heures à température ambiante, l'excès d'anhydride acétique et l'acide acétique formé au cours de la réaction sont éliminés sous pression réduite. Le produit brut est dilué avec de l'éther de méthyle et de *t*-butyle et la phase organique est lavée deux fois avec de l'eau, puis avec une solution aqueuse saturée de bicarbonate de sodium et enfin avec de la saumure. Après séchage sur sulfate de magnésium, les solvants sont évaporés et le produit brut est distillé pour donner l'acétate de 2,6-diméthyl-4-(2,2,3-triméthylcyclopent-3-ényl)cyclohex-2-ényle avec un rendement de 73%, sous forme d'un mélange de 3 stéréoisomères principaux (85%) dans un rapport de 25:60:15.

**Téb.** : 96°C / 0.067 kPa (0.5 torr)
**Profil olfactif** : légèrement boisé, faible.
Analyses RMN correspondant au dérivé attendu (à comparer avec l'alcool correspondant de l'exemple 19).

### Exemple 42: Préparation de l'acétate de 2,6-diméthyl-4-(1-phényléthyl)cyclohex-2-ènyle

L'acétate de 2,6-diméthyl-4-(1-phényléthyl)cyclohex-2-ènyle est obtenu avec un rendement de 75%, selon l'exemple 41, à partir du 2,6-diméthyl-4-(1-phényléthyl)cyclohex-2-èn-1-ol (obtenu avec un rendement de 72%, selon l'exemple 22, à partir de la 2,6-diméthyl-4-(1-phényléthyl)cyclohex-2-ènone, obtenue dans l'exemple 6).

Il s'agit d'un mélange de 5 stéréoisomères (91%) observables dans un rapport 12:14:14:25:35.

**Téb.:** 120°C /0.013 kPa (0.1 torr)
**Profil olfactif** : fleuri, miellé, pépin de citron écrasé.
**RMN-¹H (CDCl₃, 300MHz):**
δ(ppm) protons communs 0.95-1.22 (m, 1H), 1.37-2.07 (m, 2H), 2.14-2.49 (m, 1H), 2.49-2.73 (m, 1H), 4.87-5.31 (m, 1H), 7.16-7.26 (m, 3H), 7.28-7.37 (m, 2H).
lers Isomères majoritaires (protons caractéristiques): 0.89 (d, *J* = 6.54 Hz, 3H), 1.33 (d, *J* = 6.90 Hz, 3H), 1.63-1.67 (m, 3H), 5.31-5.37 (m, 1H).
2èmes Isomères majoritaires (protons caractéristiques): 0.98 (d, *J* = 6.45 Hz, 3H), 1.25 (d, *J* = 6.96 Hz, 3H), 1.55-1.59 (m, 3H), 5.65-5.69 (m, 1H).
lers Isomères minoritaires (protons caractéristiques): 0.83 (d, *J* = 6.71 Hz, 3H), 1.34 (d, *J* = 6.90 Hz, 3H), 1.59-1.62 (m, 3H), 5.82-5.89 (m, 1H).
**RMN-¹³C (CDCl₃, 75MHz):** 4 isomères observables
Isomères majoritaires: 171.21 (C(O)), 145.46 (C^{IV}), 133.87 (C^{IV}), 129.52 (CH), 128.1 (CH), 127.44 (CH), 125.89 (CH), 77.84 (CHOAc), 44.44 (CH), 42.67 (CH), 35.41 (CH₂), 35.08 (CH), 20.80 (CH₃), 19.10 (CH₃), 18.62 (CH₃), 18.14 (CH₃).
lers Isomères minoritaires (pics caractéristiques): 145.68 (C^{IV}), 133.28 (C^{IV}), 129.11 (CH), 77.84 (CHOAc), 33.73 (CH₂).
2èmes Isomères minoritaires (pics caractéristiques): 146.37 (C^{IV}), 132.39 (C^{IV}), 129.15 (CH), 72.69 (CHOAc), 29.45 (CH₂).

### Exemple 43: Préparation de l'acétate de 4-(4,4-diméthylpentan-2-yl)-2,6-diméthylcyclohex-2-ènyle

L'acétate de 4-(4,4-diméthylpentan-2-yl)-2,6-diméthylcyclohex-2-ènyle est obtenu avec un rendement de 60%, selon l'exemple 41, à partir du 4-(4,4-diméthylpentan-2-yl)-2,6-diméthylcyclohex-2-èn-1-ol (obtenu dans l'exemple 29).

Il s'agit d'un mélange de stéréoisomères (5 observés), dont 2 stéréoisomères principaux (80%) dans un rapport 71:29.

Téb.: 95°C /0.053 kPa (0.4 torr)
**Profil olfactif:** boisé, un peu cacao, assez similaire à l'alcool correspondant, mais plus faible.
**RMN-¹H (CDCl₃, 200MHz):**
δ(ppm) protons communs 0.8-0.95 (m, 15H), 0.95-1.32 (m, 2H), 1.40-1.55 (m, 2H), 1.65-1.90 (m, 1H), 1.90-2.25 (m, 1H).
Isomères majoritaires (protons caractéristiques): 1.56-1.60 (m, 3H), 2.09 (s, 3H), 5.12-5.22 (m, 1H), 5.42-5.50 (m, 1H).
Isomères minoritaires (protons caractéristiques): 1.62-1.66 (m, 3H), 2.07 (s, 3H), 5.30-5.40 (m, 2H).
**RMN-¹³C (CDCl₃, 50MHz):** 4 isomères observables
Isomères majoritaires (pics caractéristiques): 171.43 (C(O)), 133.78 & 133.39 (C^{IV}), 130.55 & 130.14 (CH), 78.37 & 78.32 (CHOAc), 47.65 & 47.49 (CH₂), 43.28 & 43.13 (CH), 32.83 & 32.43 (CH₂), 31.03 & 30.97 (C^{IV}), 29.94 (3 CH₃).
Isomères minoritaires (pics caractéristiques): 171.05 (C(O)), 132.05 & 131.77 (C^{IV}), 129.79 & 129.37 (CH), 74.09 & 73.83 (CHOAc), 48.51 & 48.19 (CH₂), 39.56 & 38.90 (CH), 31.03 & 30.97 (C^{IV}), 29.94 (3 CH₃), 29.50 & 28.78 (CH₂).

### Exemple 44: Préparation de l'acétate de 4-isopropyl-2,2,6,6-tétraméthylcyclohex-3-ènyle

L'acétate de 4-isopropyl-2,2,6,6-tétraméthylcyclohex-3-ènyle est obtenu avec un rendement de 53% sur 2 étapes (réduction selon l'exemple 37, suivie d'une estérification selon l'exemple 41), à partir de la 4-isopropyl-2,2,6,6-tétraméthylcyclohex-3-èn-1-one (obtenu dans l'exemple 17).

Téb.: 75°C /0.053 kPa (0.4 torr)
**Profil olfactif :** Boisé, humide, un peu patchouli
**RMN-¹H (CDCl₃, 200MHz):** δ 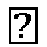 (ppm) 0.89 (s, 3H), 0.92 (s, 3H), 0.95 (s, 6H), 0.96 (s, 3H), 0.97 (s, 3H), 1.7-2.0 (m, 2H), 2.10 (s, 3H), 2.14 (hept, J = 6.82 Hz, 1H), 4.78 (s, 1H), 5.04 (m, 1H).
**RMN-¹³C (CDCl₃, 50MHz):** δ 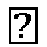 (ppm)
171.16 (C(O)), 138.52 (C^{IV}), 127.64 (CH), 82.57 (CHOAc), 39.64 (CH₂), 36.68 (C^{IV}), 34.75 (CH), 34.62 (C^{IV}), 30.89 (CH₃), 28.33 (CH₃), 23.76 (CH₃), 22.38 (CH₃), 21.25 (CH₃), 21.03 (CH₃), 20.93 (CH₃).

### Exemple 45: Préparation de l'acétate de 4-isopropyl-2,6-diméthylcyclohexyle

L'acétate de 4-isopropyl-2,6-diméthylcyclohexyle est obtenu avec un rendement de 60%, selon l'exemple 41, à partir du 4-isopropyl-2,6-diméthylcyclohexanol obtenu avec un rendement de 44%, sur 2 étapes (hydrogénation selon l'exemple 19, suivie d'une réduction de la cétone selon l'exemple 37, à partir de la cyclohexènone obtenue dans l'exemple 10).

Il s'agit d'un mélange de stéréoisomères (4 observés), dont 2 stéréoisomères principaux (82%) dans un rapport 65:35.

Téb.: 48°C /0.053 kPa (0.4 torr)
**Profil olfactif:** citrus, pamplemousse, un peu rhubarbe, puis boisé, écorce d'agrume, poussiéreux.
**RMN-¹H (CDCl₃, 200MHz):** δ 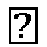 (ppm) protons communs 0.75-1.07 (m, 14H), 1.08-1.25 (m, 1H), 1.27-1.9 (m, 5H),
Isomères majoritaires (pics caractéristiques): δ(ppm) 2.05 (s, 3H), 4.98 (s, 1H). Isomères minoritaires (pics caractéristiques): δ(ppm) 2.06 (s, 3H), 4.26 (t, *J* = 10.30 Hz, 1H).
**RMN-¹³C (CDCl₃, 50MHz):** δ(ppm)
Isomères majoritaires : δ(ppm) 171.14 (C(O)), 76.11 (CHOAc), 43.38 (CH), 36.0 (CH/CH₃), 32.66 (CH), 31.91 (CH₂), 19.78 (CH₃), 18.19 (CH₃).
Isomères minoritaires : δ(ppm) 171.14 (C(O)), 82.99 (CHOAc), 42.62 (CH), 37.30 (CH/CH₃), 36.93 (CH₂), 32.23 (CH), 19.76 (CH₃), 18.50 (CH₃).

### Exemple 46: Préparation du 4-(4,4-diméthylpentan-2-yl)-1-méthoxy-2,6-diméthylcyclohex-2-ène

A une suspension de NaH (1.2 éq.) dans le THF est ajouté le 4-(4,4-diméthylpentan-2-yl)-2,6-diméthylcyclohex-2-èn-1-ol. Après 4 heures à température ambiante (fin du dégagement gazeux), le mélange est refroidi à 0°C et l'iodure de méthyle (1.2 éq.) est ajouté lentement, au goutte à goutte, le milieu réactionnel est alors chauffé à 40°C durant une nuit. Une fois la réaction terminée (suivi CPG), le milieu réactionnel est versé dans un mélange éther de méthyle et de *t*-butyle (MTBE) / HCl 10% aq. La phase aqueuse est extraite 2 fois au MTBE et les phases organiques réunies sont lavées avec une solution aqueuse à 10% de thiosulfate de sodium, puis avec une solution aqueuse saturée de bicarbonate de sodium et à la saumure. Après séchage sur sulfate de magnésium, filtration sur papier et évaporation des solvants, le produit brut est purifié par distillation sous pression réduite pour donner le 1-(4,4-diméthylpentan-2-yl)-1-méthoxy-2,6-diméthylcyclohex-2-ène avec un rendement de 57%.

Il s'agit d'un mélange de stéréoisomères (8 observés), dont 4 stéréoisomères principaux (88%) dans un rapport 18:17:33:32.

**Téb.:** 74°C /0.053 kPa (0.4 torr)
**Profil olfactif** : boisé, chocolat
**RMN-¹H (CDCl₃, 200MHz,** protons communs): δ(ppm) 0.8-0.92 (m, 13H), 0.92-1.07 (m, 3H), 1.07-1.4 (m, 2H), 1.4-1.65 (m, 2H).
Isomères majoritaires (protons caractéristiques) : 1.68-1.72 (m, 3H), 3.28 & 3.29 (s, 3H), 3.37-3.42 & 3.42-3.47 (m, 1H), 5.24-5.3 & 5.3-5.34 (m, 1H).
Isomères minoritaires (protons caractéristiques) : 1.71-1.75 (m, 3H), 3.37 & 3.38 (s, 3H), 3.57-3.67 (m, 1H), 5.34-5.38 (m, 1H).
**RMN-¹³C (CDCl₃, 50MHz):** δ(ppm)
Isomères majoritaires : 135.95 & 135.49 (C^{IV}), 130.72 & 130.09 (CH), 85.42 & 85.39 (CHOMe), 55.40 & 55.27 (OCH₃), 47.80 & 47.68 (CH₂), 43.49 & 43.32 (CH), 33.84 & 33.79 (CH), 33.65 *&* 32.97 (CH₂), 33.26 & 32.93 (CH), 30.99 (C^{IV}), 29.97 (3CH₃), 19.48 (2(CH₃)), 19.33 (2(CH₃)), 19.27 *&* 18.97 (CH₃).
Isomères minoritaires : 134.59 *&* 134.27 (C^{IV}), 127.41 & 126.91 (CH), 81.57 *&* 81.54 (CHOMe), 57.45 *&* 57.19 (OCH₃), 48.19 *&* 48.01 (CH₂), 39.01 *&* 38.50 (CH), 33.21 & 32.93 (CH), 30.99 *&* 29.36 (CH₂), 30.99 (C^{IV}), 29.97 (3CH₃), 29.26 *&* 29.21 (CH), 20.29 & 20.22 (CH₃), 19.82 & 19.27 (CH₃), 13.06 *&* 12.80 (CH₃).

## Revendications

1. Procédé de préparation d'un composé de formule (I) 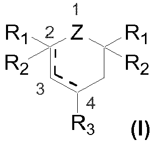 dans laquelle :
- R1 représente un méthyle ou un éthyle ;
- R2 représente indépendamment un hydrogène ou un groupement C1-C5 alkyle ou C2-C5 alcényle ;
- R3 représente un groupement alkyle ou alcényle, éventuellement substitué par un aryle, ou R3 représente un groupement alkyle cyclique ou alcényle cyclique éventuellement substitué par un ou plusieurs groupements C1-C6 alkyle, étant entendu que R3 comporte au total 3 à 10 atomes de carbone ;
- Z représente C(O) ou CR4(OR5), avec
- R4 représentant un hydrogène ou un groupement C1-C8 alkyle ou C2-C8 alcényle ;
- R5 représentant un hydrogène ou un groupement C1-C8 alkyle ou alcanoyle, ou C2-C8 alcényle ou alcénoyle ;
sachant qu'une double liaison est présente ou absente, sur le cycle et que lorsqu'elle est présente, elle est
- soit en position 2-3 et R2 est absent en position 2,
- soit en position 3-4 et R2 est présent en position 2 et est tel que défini précédemment,
**caractérisé en ce que** ledit procédé comprend les étapes suivantes :
i) réaction d'un α-méthylène-aldéhyde, en présence d'une base, sur une cétone symétrique pour obtenir un composé de formule (la), 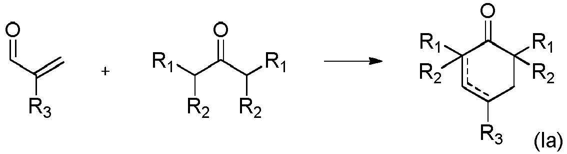 dans laquelle R1, et R3 sont tels que définis précédemment, R2 est un hydrogène, et une double liaison en 2-3 ou 3-4 est présente sur le cycle et, cette réaction étant suivie éventuellement par les étapes ii), et/ou iii), et/ou iv),
ii) réaction de mono- ou bis-alkylation afin d'obtenir un composé de formule (la) dans laquelle R2 est un groupement C1-C5 alkyle ou C2-C5 alcényle ;
iii) conversion de la fonction Z = C(O) du composé obtenu à l'étape précédente en une fonction Z=CR4(OR5), R4 et R5 étant tels que définis précédemment ;
iv) réduction de la double liaison en 2-3 ou 3-4 présente sur le cycle du composé obtenu à l'étape précédente, l'étape iv) pouvant être réalisée après l'une quelconque des étapes i), ii), ou iii).

2. Procédé selon la revendication 1 **caractérisé en ce que** l'étape iii) comprend une étape iii.a) de réduction de la fonction cétone du composé obtenu aux étapes i), ii) ou iv) pour obtenir un composé de formule (Ib) : 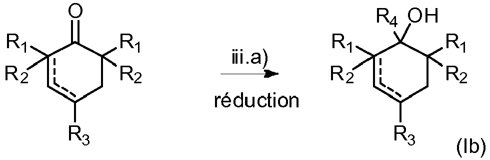
avec R1, R2, R3, tels que définis précédemment à l'étape i) et/ou ii) à la revendication 1,
R4 représente un hydrogène ou un groupement C1-C8 alkyle ou C2-C8 alcényle, et
la double liaison en 2-3 ou 3-4 étant absente dans le cas où l'étape iv) est réalisée avant l'étape iii.a).

3. Procédé selon la revendication précédente **caractérisé en ce que** la réaction de réduction se fait par addition d'un organomagnésien ou d'un hydrure métallique.

4. Procédé selon la revendication 1 **caractérisé en ce que** l'étape iii) comprend, outre l'étape iii.a) de réduction telle que définie à la revendication 2, une étape iii.b) d'alkylation de la fonction alcool du composé (Ib) obtenu à l'étape iii.a), pour obtenir un composé de formule (Ic) : 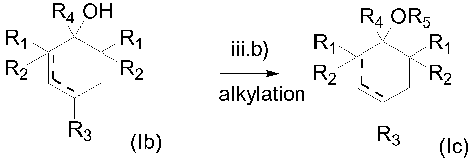
avec R1, R2, R3, R4 tels que définis à la revendication 2,
R5 représente un alkyle ou un alcényle, et
la double liaison en 2-3 ou 3-4 étant absente dans le cas où l'étape iv) est réalisée avant l'étape iii.b).

5. Procédé selon la revendication précédente **caractérisé en ce que** l'étape d'alkylation iii.b) se fait par addition d'un halogénure d'alkyle.

6. Procédé selon la revendication 1, **caractérisé en ce que** l'étape iii) comprend, outre l'étape iii.a) telle que définie à la revendication 2, une étape iii.c) d'estérification de la fonction alcool du composé (Ib) obtenu à l'étape iii.a), pour obtenir un composé (Id) 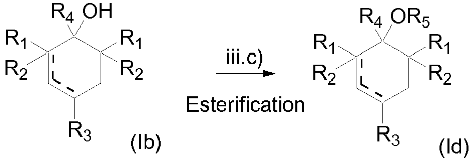
avec R1, R2, R3, R4 tels que définis à la revendication 2, R5 représente un alcanoyle ou un alcénoyle, et
la double liaison en 2-3 ou 3-4 étant absente dans le cas où l'étape iv) est réalisée avant l'étape iii.c).

7. Procédé selon la revendication précédente **caractérisé en ce que** l'étape d'estérification iii.c) se fait par addition d'un chlorure d'acyle ou d'un anhydride.

8. Composé de formule générale (II) suivante : 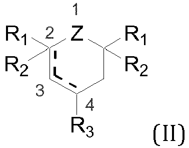 dans laquelle :
- R1 représente un méthyle ou un éthyle ;
- R2 représente indépendamment un hydrogène ou un groupement C1-C5 alkyle ou C2-C5 alcényle ;
- R3 représente un groupement alkyle ou alcényle, éventuellement substitué par un aryle, ou R3 représente un groupement alkyle cyclique comprenant cinq à six atomes de carbone ou alcényle cyclique, éventuellement substitué par un ou plusieurs groupements C1-C6 alkyle, étant entendu que R3 comporte au total 7 à 10 atomes de carbone ;
- Z représente C(O) ou CR4(OR5), avec
- R4 représentant un hydrogène ou un groupement C1-C8 alkyle ou C2-C8 alcényle ;
- R5 représentant un hydrogène ou un groupement C1-C8 alkyle ou alcanoyle, ou C2-C8 alcényle ou alcénoyle ;
sachant qu'une double liaison est présente ou absente, sur le cycle et que lorsqu'elle est présente, elle est
- soit en position 2-3 et R2 est absent en position 2,
- soit en position 3-4 et R2 est présent en position 2 et est tel que défini précédemment;
ledit composé étant sous forme d'un stéréoisomère ou d'un mélange de stéréoisomères, ou d'un mélange racémique.

9. Composé selon la revendication précédente **caractérisé en ce que** R3 est soit un groupement cyclopentyle substitué par un ou plusieurs groupements alkyles, soit un groupement cyclopentényle susbtitué par un ou plusieurs groupements alkyles.

10. Composé selon la revendication 8 **caractérisé en ce que** R3 est un groupement alkyle ou alcényle, éventuellement substitué par un aryle.

11. Composé selon l'une des revendications 8 à 10 **caractérisé en ce que** Z=C(O).

12. Composé selon l'une des revendications 8 à 11 **caractérisé en ce que** Z=CR4(OR5) avec R4 représentant un hydrogène ou un groupement C1-C8 alkyle ou C2-C8 alcényle, et R5 représentant un hydrogène.

13. Composition **caractérisée en ce qu'**elle comprend au moins un composé de formule générale (II) telle que définie dans l'une des revendications 8 à 12, sous forme d'un stéréoisomère ou d'un mélange de stéréoisomères, ou d'un mélange racémique.

14. Composition selon la revendication précédente **caractérisée en ce qu'**elle comprend en outre au moins une autre substance odorante.

15. Composition selon l'une des revendications 13 ou 14 **caractérisé en ce que** le composé de formule (II) est présent dans une concentration comprise entre 0.1 et 99 % en poids par rapport au poids total de la composition, notamment entre 0,1 et 30%.

16. Utilisation en tant qu'agent fragrant d'un composé de formule générale (III)
suivante : 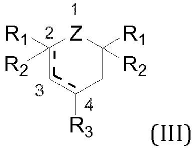 dans laquelle :
- R1 représente un méthyle ou un éthyle ;
- R2 représente indépendamment un hydrogène ou un groupement C1-C5 alkyle ou C2-C5 alcényle ;
- R3 représente un groupement alkyle ou alcényle, éventuellement substitué par un aryle, ou R3 représente un groupement alkyle cyclique comprenant cinq à six atomes de carbone ou alcényle cyclique, éventuellement substitué par un ou plusieurs groupements C1-C6 alkyle, étant entendu que R3 comporte au total 3 à 10 atomes de carbone et qu'il comporte au moins une insaturation lorsqu'il compte 5 à 6 atomes de carbone ;
- Z représente C(O) ou CR4(OR5), avec
- R4 représentant un hydrogène ou un groupement C1-C8 alkyle ou C2-C8 alcényle ;
- R5 représentant un hydrogène ou un groupement C1-C8 alkyle ou alcanoyle, ou C2-C8 alcényle ou alcénoyle ;
sachant qu'une double liaison est présente ou absente, sur le cycle et que lorsqu'elle est présente, elle est
- soit en position 2-3 et R2 est absent en position 2,
- soit en position 3-4 et R2 est présent en position 2 et est tel que défini précédemment.

17. Utilisation d'un composé de formule (III) tel que défini en revendication 16, en tant qu'agent masquant d'une odeur ou en tant qu'agent neutralisateur d'une odeur.

18. Utilisation selon l'une des revendications 16 ou 17 d'au moins un composé de formule (III) seul ou en combinaison avec au moins un autre ingrédient aromatisant ou parfumant, et/ou au moins un solvant, et/ou au moins un adjuvant.

19. Utilisation selon l'une des revendications 16 à 18 pour conférer, modifier ou renforcer les propriétés organoleptiques d'une substance, d'une composition ou d'un article.

## Patentansprüche

1. Ein Zubereitungsverfahren einer Verbindung von Formel (I) 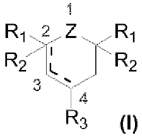 wobei:
- R1 ein Methyl oder ein Ethyl darstellt;
- R2 unabhängig Wasserstoff oder eine C1-C5-Alkyl- oder C2-C5-Alkenylgruppe darstellt;
- R3 eine Alkyl- oder Alkenylgruppe darstellt, gegebenenfalls substituiert durch ein Aryl, oder R3 eine cyclische Alkylgruppe oder cyclische Alkenylgruppe darstellt, gegebenenfalls substituiert durch eine oder mehrere C1-C6-Alkylgruppen, wobei R3 insgesamt 3 bis 10 Kohlenstoffatome umfasst;
- Z C(O) oder CR4(OR5) darstellt, wobei
- R4 Wasserstoff oder eine C1-C8-Alkyl- oder C2-C8-Alkenylgruppe darstellt;
- R5 Wasserstoff oder eine C1-C8-Alkyl- oder -Alkanoylgruppe oder eine C2-C8-Alkenyl- oder -Alkenoylgruppe darstellt;
wobei eine Doppelbindung am Ring vorhanden oder abwesend ist und sie, wenn sie vorhanden ist,
- entweder an Position 2-3 vorliegt und R2 an Position 2 abwesend ist
- oder an Position 3-4 vorliegt und R2 an Position 2 vorhanden ist und wie vorher definiert ist,
**dadurch gekennzeichnet, dass** das Verfahren die folgenden Schritte beinhaltet:
i) Reaktion eines α-Methylenaldehyds in Gegenwart einer Base an einem symmetrischen Keton, um eine Verbindung von Formel (la) zu erhalten, 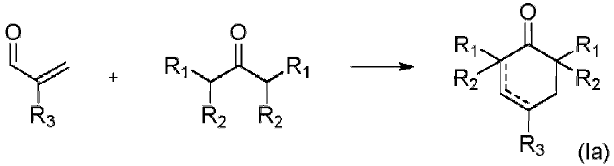 wobei R1 und R3 wie vorher definiert sind, R2 Wasserstoff ist und eine Doppelbindung an 2-3 oder 3-4 an dem Ring vorliegt und diese Reaktion gegebenenfalls gefolgt ist von den Schritten ii) und/oder iii) und/oder iv),
ii) Mono- oder Bisalkylierungsreaktion zum Erhalten einer Verbindung von Formel (la), in der R2 eine C1-C5-Alkyl- oder C2-C5-Alkenylgruppe ist;
iii) Umwandlung der Funktion Z = C(O) der im vorhergehenden Schritt erhaltenen Verbindung in eine Funktion Z = CR4(OR5), wobei R4 und R5 wie vorher definiert sind;
iv) Reduktion der an 2-3 oder 3-4 des Rings der im vorhergehenden Schritt erhaltenen Verbindung vorhandenen Doppelbindung, wobei Schritt iv) nach einem beliebigen der Schritte i), ii) oder iii) durchgeführt werden kann.

2. Verfahren gemäß Anspruch 1, **dadurch gekennzeichnet, dass** der Schritt iii) einen Schritt iii.a) der Reduktion der Ketonfunktion der in Schritten i), ii) oder iv) erhaltenen Verbindung zum Erhalten einer Verbindung von Formel (Ib) beinhaltet: 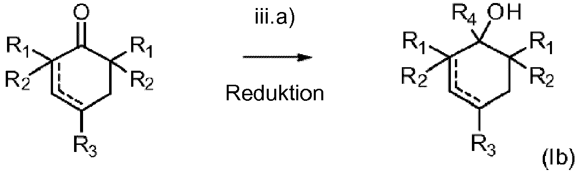 wobei R1, R2, R3 wie vorher in Schritt i) und/oder ii) in Anspruch 1 definiert sind, wobei R4 Wasserstoff oder eine C1-C8-Alkyl- oder C2-C8-Alkenylgruppe darstellt und die Doppelbindung an 2-3 oder 3-4 in dem Fall, in dem Schritt iv) vor Schritt iii.a) durchgeführt wird, abwesend ist.

3. Verfahren gemäß dem vorhergehenden Anspruch, **dadurch gekennzeichnet, dass** die Reduktionsreaktion durch Hinzufügen eines Magnesiumorganyls oder eines Metallhydrids stattfindet.

4. Verfahren gemäß Anspruch 1, **dadurch gekennzeichnet, dass** Schritt iii) zusätzlich zum Reduktionsschritt iii.a), wie gemäß Anspruch 2 definiert, einen Schritt iii.b) der Alkylierung der Alkoholfunktion der in Schritt iii.a) erhaltenen Verbindung (Ib) beinhaltet, um eine Verbindung von Formel (Ic) zu erhalten: 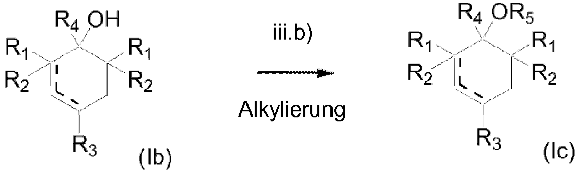
wobei R1, R2, R3, R4 wie gemäß Anspruch 2 definiert sind,
wobei R5 ein Alkyl oder ein Alkenyl darstellt und
die Doppelbindung an 2-3 oder 3-4 in dem Fall, in dem Schritt iv) vor Schritt iii.b) durchgeführt wird, abwesend ist.

5. Verfahren gemäß dem vorhergehenden Anspruch, **dadurch gekennzeichnet, dass** der Alkylierungsschritt iii.b) durch Hinzufügen eines Alkylhalogenids stattfindet.

6. Verfahren gemäß Anspruch 1, **dadurch gekennzeichnet, dass** Schritt iii) zusätzlich zu Schritt iii.a), wie gemäß Anspruch 2 definiert, einen Schritt iii.c) der Veresterung der Alkoholfunktion von Verbindung (Ib), erhalten in Schritt iii.a), beinhaltet,
um eine Verbindung (Id) zu erhalten 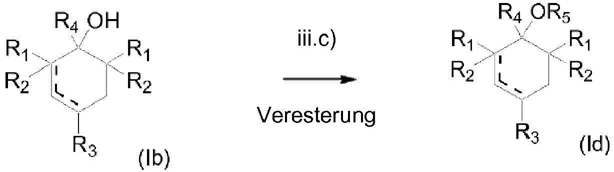
wobei R1, R2, R3, R4 wie gemäß Anspruch 2 definiert sind, wobei R5 ein Alkanoyl oder ein Alkenoyl darstellt und
die Doppelbindung an 2-3 oder 3-4 abwesend ist in dem Fall, in dem Schritt iv) vor Schritt iii.c) durchgeführt wird.

7. Verfahren gemäß dem vorhergehenden Anspruch, **dadurch gekennzeichnet, dass** der Veresterungsschritt iii.c) durch Hinzufügen eines Acylchlorids oder eines Anhydrids stattfindet.

8. Eine Verbindung der folgenden allgemeinen Formel (II): 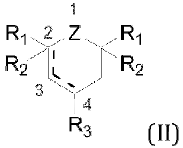 wobei:
- R1 ein Methyl oder ein Ethyl darstellt;
- R2 unabhängig Wasserstoff oder eine C1-C5-Alkyl- oder C2-C5-Alkenylgruppe darstellt;
- R3 eine Alkyl- oder Alkenylgruppe darstellt, gegebenenfalls substituiert durch ein Aryl, oder R3 eine cyclische Alkylgruppe, beinhaltend fünf bis sechs Kohlenstoffatome, oder eine cyclische Alkenylgruppe, gegebenenfalls substituiert durch eine oder mehrere C1-C6-Alkylgruppen, darstellt, wobei R3 insgesamt 7 bis 10 Kohlenstoffatome umfasst;
- Z C(O) oder CR4(OR5) darstellt, wobei
- R4 Wasserstoff oder eine C1-C8-Alkyl- oder C2-C8-Alkenylgruppe darstellt;
- R5 Wasserstoff oder eine C1-C8-Alkyl- oder -Alkanoylgruppe oder eine C2-C8-Alkenyl- oder -Alkenoylgruppe darstellt;
wobei eine Doppelbindung am Ring vorhanden oder abwesend ist und sie, wenn sie vorhanden ist,
- entweder an Position 2-3 vorliegt und R2 an Position 2 abwesend ist
- oder an Position 3-4 vorliegt und R2 an Position 2 vorhanden ist und wie vorher definiert ist;
wobei die Verbindung in Form eines Stereoisomers oder einer Mischung von Stereoisomeren oder einer racemischen Mischung vorliegt.

9. Verbindung gemäß dem vorhergehenden Anspruch, **dadurch gekennzeichnet, dass** R3 entweder eine durch eine oder mehrere Alkylgruppen substituierte Cyclopentylgruppe oder eine durch eine oder mehrere Alkylgruppen substituierte Cyclopentenylgruppe ist.

10. Verbindung gemäß Anspruch 8, **dadurch gekennzeichnet, dass** R3 eine Alkyl- oder Alkenylgruppe ist, gegebenenfalls durch ein Aryl substituiert.

11. Verbindung gemäß einem der Ansprüche 8 bis 10, **dadurch gekennzeichnet, dass** Z = C(O).

12. Verbindung gemäß einem der Ansprüche 8 bis 11, **dadurch gekennzeichnet, dass** Z = CR4(OR5), wobei R4 Wasserstoff oder eine C1-C8-Alkyl- oder C2-C8-Alkenylgruppe darstellt und R5 Wasserstoff darstellt.

13. Eine Zusammensetzung, **dadurch gekennzeichnet, dass** sie mindestens eine Verbindung von allgemeiner Formel (II), wie gemäß einem der Ansprüche 8 bis 12 definiert, in Form eines Stereoisomers oder einer Mischung von Stereoisomeren oder einer racemischen Mischung beinhaltet.

14. Zusammensetzung gemäß dem vorhergehenden Anspruch, **dadurch gekennzeichnet, dass** sie des Weiteren mindestens eine andere wohlriechende Substanz beinhaltet.

15. Zusammensetzung gemäß einem der Ansprüche 13 oder 14, **dadurch gekennzeichnet, dass** die Verbindung von Formel (II) bezogen auf das Gesamtgewicht der Zusammensetzung in einer Konzentration im Bereich zwischen 0,1 und 99 Gewichts-%, insbesondere zwischen 0,1 und 30 %, vorhanden ist.

16. Eine Verwendung einer Verbindung der folgenden allgemeinen Formel (III) als Duftstoff: 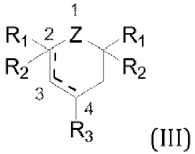 wobei:
- R1 ein Methyl oder ein Ethyl darstellt;
- R2 unabhängig Wasserstoff oder eine C1-C5-Alkyl- oder C2-C5-Alkenylgruppe darstellt;
- R3 eine Alkyl- oder Alkenylgruppe darstellt, gegebenenfalls substituiert durch ein Aryl, oder R3 eine cyclische Alkylgruppe, beinhaltend fünf bis sechs Kohlenstoffatome, oder eine cyclische Alkenylgruppe, gegebenenfalls substituiert durch ein oder mehrere C1-C6-Alkylgruppen, darstellt, wobei R3 insgesamt 3 bis 10 Kohlenstoffatome umfasst und wobei er mindestens eine Ungesättigtheit umfasst, wenn er 5 bis 6 Kohlenstoffatome zählt;
- Z C(O) oder CR4(OR5) darstellt, wobei
- R4 Wasserstoff oder eine C1-C8-Alkyl- oder C2-C8-Alkenylgruppe darstellt;
- R5 Wasserstoff oder eine C1-C8-Alkyl- oder -Alkanoylgruppe oder eine C2-C8-Alkenyl- oder -Alkenoylgruppe darstellt;
wobei eine Doppelbindung am Ring vorhanden oder abwesend ist und sie, wenn sie vorhanden ist,
- entweder an Position 2-3 vorliegt und R2 an Position 2 abwesend ist
- oder an Position 3-4 vorliegt und R2 an Position 2 vorhanden ist und wie vorher definiert ist.

17. Verwendung einer Verbindung von Formel (III), wie in Anspruch 16 definiert, als Maskierungsmittel eines Geruchs oder als ein Mittel zum Neutralisieren des Geruchs.

18. Verwendung gemäß einem der Ansprüche 16 oder 17 von mindestens einer Verbindung von Formel (III), alleine oder in Kombination mit mindestens einem anderen aromatisierenden oder parfümierenden Inhaltsstoff und/oder mindestens einem Lösemittel und/oder mindestens einem Adjuvans.

19. Verwendung gemäß einem der Ansprüche 16 bis 18, um die organoleptischen Eigenschaften einer Substanz, einer Zusammensetzung oder eines Artikels zu verleihen, zu modifizieren oder zu verstärken.

## Claims

1. Method of preparation of a compound of formula (I) 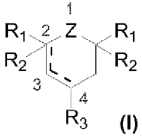 in which:
- R1 represents a methyl or an ethyl;
- R2 represents independently a hydrogen or a C1-C5 alkyl or C2-C5 alkenyl group;
- R3 represents an alkyl or alkenyl group, optionally substituted by an aryl, or R3 represents a cyclic alkyl or cyclic alkenyl group, optionally substituted by one or more C1-C6 alkyl groups, it being understood that R3 includes in total 3 to 10 carbon atoms;
- Z represents C(O) or CR4(OR5), with
- R4 representing a hydrogen or a C1-C8 alkyl or C2-C8 alkenyl group;
- R5 representing a hydrogen or a C1-C8 alkyl or alkanoyl or C2-C8 alkenyl or alkenoyl group;
knowing that a double bond is present or absent in the ring and that, when it is present, it is
- either in position 2-3 and R2 is absent in position 2,
- or in position 3-4 and R2 is present in position 2 and is as defined above, **characterised in that** said method comprises the following steps:
i) reaction of an α-methylene aldehyde, in the presence of a base, with a symmetrical ketone to obtain a compound of formula (Ia), 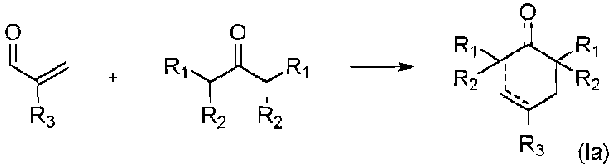 in which R1 and R3 are as defined above, R2 is a hydrogen and a double bond is present at 2-3 or 3-4 in the ring and this reaction being optionally followed by steps ii), and/or iii), and/or iv),
ii) mono- or bis-alkylation reaction in order to obtain a compound of formula (Ia) in which R2 is a C1-C5 alkyl or C2-C5 alkenyl group;
iii) conversion of the Z = C(O) function of the compound obtained in the preceding step into a Z=CR4(OR5) function, R4 and R5 being as defined above;
iv) reduction of the double bond at 2-3 or 3-4 present in the ring of the compound obtained in the preceding step, step iv) being able to be performed after any one of steps i), ii), or iii).

2. Method according to Claim 1 **characterised in that** step iii) comprises a step iii.a) of reduction of the ketone function of the compound obtained in steps i), ii) or iv) to obtain a compound of formula (Ib): 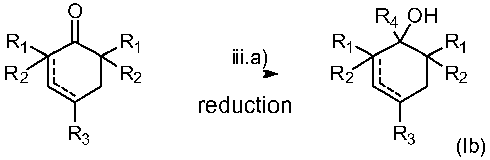
with R1, R2, R3 as defined above in step i) and/or ii) in Claim 1,
R4 represents a hydrogen or a C1-C8 alkyl or C2-C8 alkenyl group, and
the double bond at 2-3 or 3-4 being absent in the case in which step iv) is performed before step iii.a).

3. Method according to the preceding Claim **characterised in that** the reduction reaction is performed by addition of an organomagnesium or of a metallic hydride.

4. Method according to Claim 1 **characterised in that** step iii) comprises, in addition to step iii.a) of reduction as defined in claim 2, a step iii.b) of alkylation of the alcohol function of the compound (Ib) obtained in step iii.a), to obtain a compound of formula (Ic): 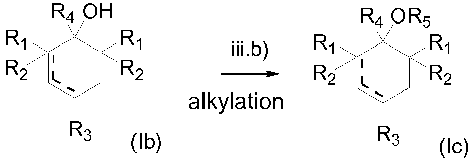
with R1, R2, R3, R4 as defined in Claim 2,
R5 represents an alkyl or an alkenyl, and
the double bond at 2-3 or 3-4 being absent in the case in which step iv) is performed before step iii.b).

5. Method according to the preceding Claim **characterised in that** the alkylation step iii.b) is performed by addition of an alkyl halide.

6. Method according to Claim 1, **characterised in that** step iii) comprises, in addition to step iii.a) as defined in Claim 2, a step iii.c) of esterification of the alcohol function of the compound (Ib) obtained in step iii.a), to obtain a compound (Id) 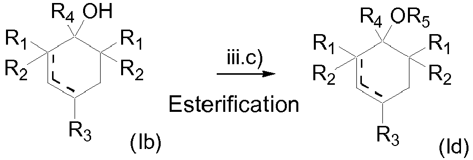 with R1, R2, R3, R4 as defined in Claim 2, R5 represents an alkanoyl or an alkenoyl, and the double bond at 2-3 or 3-4 being absent in the case in which step iv) is performed before step iii.c).

7. Method according to the preceding claim **characterised in that** the esterification step iii.c) is performed by addition of an acyl chloride or of an anhydride.

8. Compound of the following general formula (II): 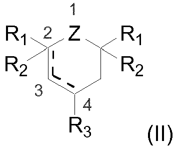 in which:
- R1 represents a methyl or an ethyl;
- R2 represents independently a hydrogen or a C1-C5 alkyl or C2-C5 alkenyl group;
- R3 represents an alkyl or alkenyl group, optionally substituted by an aryl, or R3 represents a cyclic alkyl group comprising five to six carbon atoms or cyclic alkenyl group, optionally substituted by one or more C1-C6 alkyl groups, it being understood that R3 includes in total 7 to 10 carbon atoms;
- Z represents C(O) or CR4(OR5), with
- R4 representing a hydrogen or a C1-C8 alkyl or C2-C8 alkenyl group;
- R5 representing a hydrogen or a C1-C8 alkyl or alkanoyl or C2-C8 alkenyl or alkenoyl group;
knowing that a double bond is present or absent in the ring and that, when it is present, it is
- either in position 2-3 and R2 is absent in position 2,
- or in position 3-4 and R2 is present in position 2 and is as defined above;
said compound being in the form of a stereoisomer or of a mixture of stereoisomers, or of a racemic mixture.

9. Compound according to the preceding claim **characterised in that** R3 is either a cyclopentyl group substituted by one or more alkyl groups, or a cyclopentenyl group substituted by one or more alkyl groups.

10. Compound according to claim 8 **characterised in that** R3 is an alkyl or alkenyl group, optionally substituted by an aryl.

11. Compound according to one of claims 8 to 10 **characterised in that** Z=C(O).

12. Compound according to one of claims 8 to 11 **characterised in that** Z=CR4(OR5) with R4 representing a hydrogen or a C1-C8 alkyl or C2-C8 alkenyl group, and R5 representing a hydrogen.

13. Composition **characterised in that** it comprises at least one compound of general formula (II) as defined in one of claims 8 to 12, in the form of a stereoisomer or of a mixture of stereoisomers, or of a racemic mixture.

14. Composition according to the preceding claim **characterised in that** it comprises in addition at least one other fragrancing substance.

15. Composition according to one of claims 13 or 14 **characterised in that** the compound of formula (II) is present in a concentration of between 0.1 and 99% by weight relative to the total weight of the composition, particularly between 0.1 and 30%.

16. Use as a fragrancing agent of a compound of the following general formula (III): 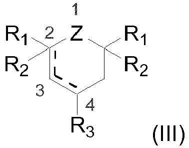 in which:
- R1 represents a methyl or an ethyl;
- R2 represents independently a hydrogen or a C1-C5 alkyl or C2-C5 alkenyl group;
- R3 represents an alkyl or alkenyl group, optionally substituted by an aryl, or R3 represents a cyclic alkyl group comprising five to six carbon atoms or cyclic alkenyl group, optionally substituted by one or more C1-C6 alkyl groups, it being understood that R3 includes in total 3 to 10 carbon atoms and that it includes at least one unsaturation when it contains 5 to 6 carbon atoms;
- Z represents C(O) or CR4(OR5), with
- R4 representing a hydrogen or a C1-C8 alkyl or C2-C8 alkenyl group;
- R5 representing a hydrogen or a C1-C8 alkyl or alkanoyl or C2-C8 alkenyl or alkenoyl group;
knowing that a double bond is present or absent in the ring and that when it is present, it is
- either in position 2-3 and R2 is absent in position 2,
- or in position 3-4 and R2 is present in position 2 and is as defined above.

17. Use of a compound of formula (III) as defined in claim 16, as an agent masking an odour or as an agent neutralising an odour.

18. Use according to one of claims 16 or 17 of at least one compound of formula (III) alone or in combination with at least one other aromatising or perfuming ingredient, and/or at least one solvent, and/or at least one adjuvant.

19. Use according to one of claims 16 to 18 to confer, modify or strengthen the organoleptic properties of a substance, of a composition or of an article.
